# EUROPEAN PATENT APPLICATION

(11) **EP 1 175 900 A2**
(43) Date of publication of application: **30.01.2002**
(21) Application number: 01306353.2
(22) Date of filing: 24.07.2001
(51) Int. Cl.: A61K 31/12, A61K 31/13, A61K 31/16, A61K 31/33, A61P 5/00

(54) **Use of growth hormone secretagogues for improvement of fucnctional health status**

(30) Priority: 27.07.2000 US 221236 P
(71) Applicant: Pfizer Products Inc., Groton, Connecticut 06340 (US)
(72) Inventor: Landschulz, William Harras, Groton, Connecticut 06340 (US); Petrie, Charles David, Groton, Connecticut 06340 (US)
(74) Representative: Hayles, James Richard

(57) **Abstract**

This invention is directed to methods for improving functional health status in a patient in need thereof which comprises administering a growth hormone secretagogue, a prodrug thereof or a pharmaceutically acceptable salt of said secretagogue or said prodrug. More preferably, the present invention provides such methods wherein the growth hormone secretagogue is a compound of Formula I: 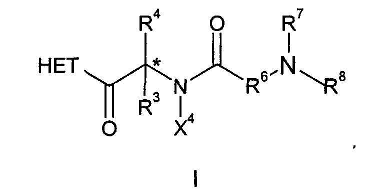 a prodrug thereof or a pharmaceutically acceptable salt of said secretagogue or said prodrug.

## Description

### FIELD OF THE INVENTION

The present invention provides methods of using growth hormone secretagogues, prodrugs thereof and pharmaceutically acceptable salts of said secretagogues and said prodrugs, for improvement in the functional health status of patients in need thereof. More specifically, the present invention provides such methods wherein the growth hormone secretagogues are certain compounds of Formula I below.

### BACKGROUND OF THE INVENTION

Patients receiving exogenous growth hormone have reported enhanced energy levels and an improved sense of well-being; however, there exists a paucity of objective data from placebo-controlled, randomized clinical trials to support such claims (S.P. McKenna and L.C. Doward, PharmacoEconomics, 6(5):434-441 (1994)). On the other hand, growth hormone-deficient adults suffer from a recognized GH deficiency neuropsychiatric syndrome which can be successfully treated, in selected adult GHD patients, by administration of recombinant growth hormone (G. McGauley et al., Horm. Res., 45: 34-37 (1996); P.V. Carroll et al., European Journal of Endocrinology, 137: 146-153 (1997); and J. O. L. Jorgensen et al., Horm. Res. 42: 235-241 (1994)).

Growth hormone, which is secreted from the pituitary, stimulates growth of all tissues of the body that are capable of growing. In addition, growth hormone is known to have the following effects on the metabolic processes of the body: (1) increased rate of protein synthesis in all cells of the body; (2) decreased rate of carbohydrate utilization in cells of the body; and (3) increased mobilization of free fatty acids and use of fatty acids for energy. As is known to those skilled in the art, the known and potential uses of growth hormone are many and varied. See "Human Growth Hormone," Strobel and Thomas, Pharmacological Reviews, 46, pg. 1-34 (1994). Also, these varied uses of growth hormone are summarized in International Patent Application, Publication Number WO 97/24369.

Various ways are known to release growth hormone (see Recent Progress in Hormone Research, vol. 52, pp. 215-245 (1997); and Front. Horm. Res., Basel, Karger, vol. 24, pp. 152-175 (1999)). For example, chemicals such as arginine, L-3,4-dihydroxyphenylalanine (L-DOPA), glucagon, vasopressin, and insulin induced hypoglycemia, as well as activities such as sleep and exercise, indirectly cause growth hormone to be released from the pituitary by acting in some fashion on the hypothalamus perhaps either to decrease somatostatin secretion or to increase secretion of growth hormone releasing factor (GRF) or ghrelin (see Nature, vol. 402, pp. 656-660 (9 December 1999)), or all of these.

In cases where increased levels of growth hormone were desired, the problem was generally solved by providing exogenous growth hormone or by administering GRF, IGF-I or a peptidyl compound which stimulated growth hormone production and/or release. In any case, the peptidyl nature of the compound necessitated that it be administered by injection. Initially, the source of growth hormone was the extraction of the pituitary glands of cadavers. This resulted in a very expensive product and carried with it the risk that a disease associated with the source of the pituitary gland could be transmitted to the recipient of the growth hormone. Recombinant growth hormone has become available which, while no longer carrying any such risk of disease transmission, it is still a very expensive product which must be given by injection. In addition, administration of exogenous growth hormone may result in side-effects, including edema, and does not correlate with the pulsatile release seen in the endogenous release of growth hormone.

Certain compounds have been developed which stimulate the release of endogenous growth hormone. Peptides which are known to stimulate the release of endogenous growth hormone include growth hormone releasing hormone and its analogs, the growth hormone releasing peptides, GHRP-6 and GHRP-1 (described in U.S. Patent No. 4,411,890; International Patent Application, Publication No. WO 89/07110; and International Patent Application, Publication No. WO 89/07111), and GHRP-2 (described in International Patent Application, Publication No. WO 93/04081), as well as hexarelin (J. Endocrinol. Invest., 15 (Suppl. 4): 45 (1992)). Other compounds possessing growth hormone secretagogue activity are disclosed in the following International Patent Applications (listed by Publication Nos.), issued U.S. Patents and published European Patent Applications, which are incorporated herein by reference: WO 98/46569, WO 98/51687, WO 98/58947, WO 98/58949, WO 98/58950, WO 99/08697, WO 99/09991, WO 95/13069, U.S. 5,492,916, U.S. 5,494,919, WO 95/14666, WO 94/19367, WO 94/13696, WO 94/11012, U.S. 5,726,319, WO 95/11029, WO 95/17422, WO 95/17423, WO 95/34311, WO 96/02530, WO 96/22996, WO 96/22997, WO 96/24580, WO 96/24587, U.S. 5,559,128, WO 96/32943, WO 96/33189, WO 96/15148, WO 96/38471, WO 96/35713, WO 97/00894, WO 97/07117, WO 97/06803, WO 97/11697, WO 97/15573, WO 97/22367, WO 97/23508, WO 97/22620, WO 97/22004, WO 97/21730, WO 97/24369, U.S. 5,663,171, WO 97/34604, WO 97/36873, WO 97/40071, WO 97/40023, WO 97/41878, WO97/41879, WO 97/46252, WO 97/44042, WO 97/38709, WO 98/03473, WO 97/43278, U.S. 5,721,251, U.S. 5,721,250, WO 98/10653, U.S. 5,919,777, U.S. 5,830,433 and EP 0995748.

In addition, the following growth hormone secretagogues are known in the art: MK-0677, L-162752 and L-163022 (Merck); NN703 and ipamorelin (Novo Nordisk); hexarelin (Pharmacia); GPA-748 (KP102, GHRP-2) (American Home Products); and LY444711 (Eli Lilly). The following agents that stimulate GH release via GHRH/GRF receptor (including GHRH/GRF derivatives, analogs and mimetics) are known in the art: Geref (Ares/Serono); GHRH (1-44) (BioNebraska); Somatorelin (GRF 1-44) (Fujisawa/ICN); and ThGRF (Theratechnologies).

Endocrine Reviews 18(5): 621-645 (1997) provides an overview of peptidomimetic regulation of growth hormone secretion by growth hormone secretagogues. Horm. Res. 1999; 51(suppl 3):16-20 (1999), examines the clinical and experimental effects of growth hormone secretagogues on various organ systems. Drug Discovery Today, Vol. 4, No. 11, November 1999; and TEM Vol. 10, No. 1, 1999, discloses potential therapeutic applications of growth hormone secretagogues, including their use in treating growth hormone disorders such as growth hormone deficiency (GHD), age-related conditions, obesity and catabolic conditions, and their use in sleep enhancement.

International Patent Applications, Publication Nos. WO 97/24369 and WO 98/58947 disclose that certain growth hormone secretagogues are useful for the treatment or prevention of osteoporosis, congestive heart failure, frailty associated with aging, obesity, accelerating bone fracture repair, attenuating protein catabolic response after a major operation, reducing cachexia and protein loss due to chronic illness, accelerating wound healing, accelerating the recovery of burn patients, accelerating the recovery of patients having undergone major surgery, improving muscle strength, mobility, maintenance of skin thickness, metabolic homeostasis or renal homeostasis. Published European patent application 0995748 discloses that certain dipeptide growth hormone secretagogues are useful for the treatment or prevention of musculoskeletal frailty, including osteoporosis.

The administration of a growth hormone secretagogue is also known to enhance the quality of sleep, which is disclosed in International Patent Application, Publication No. WO 97/24369. Commonly assigned U.S. nonprovisional patent application 09/290985, filed 13 April 1999, discloses pharmaceutical compositions comprising certain β₃ adrenergic agonists and growth hormone secretagogues or growth hormone, and their use for treating diabetes, obesity, hyperglycemia, frailty associated with obesity or frailty associated with aging, and for enhancing the quality of sleep in a mammal. International Patent Application, Publication No. WO 98/58949, discloses the treatment of insulin resistance with certain growth hormone secretagogues.

S.P. McKenna and L.C. Doward, PharmacoEconomics, 6(5):434-441 (1994), disclose the results of recent clinical studies, which suggest that replacement of growth hormone does have a positive effect on well-being.

G. McGauley et al., Horm. Res., 45:34-37 (1996), conclude that there is increasingly convincing literature indicating that growth hormone treatment, in selected adult GHD patients, can lead to an improved quality of Ifie.

M.E. Wallymahmed et al., Clinical Endocrinology, 44: 403-411 (1996), disclose a health-related quality of life (QOL) model specifically designed for use in adults with growth hormone deficiency and to assess the impact of future growth hormone replacement therapy.

P.V. Carroll et al., European Journal of Endocrinology, 137: 146-153 (1997), disclose that growth hormone replacement in GH-deficient adults was associated with significant improvements in self-perceived well-being as well as changes in body composition and other variables.

International Patent Application, Publication No. WO 00/12407, discloses that a growth hormone secretagogue is useful for enhancing the return of patients to independent living status following acute deconditioning such as that which may result from immobilization, surgery, or major injury such as hip fracture.

Journal of Orthopaedic Research 15:519:527 (1997) discloses that a growth hormone secretagogue, MK-0677, elevated levels of serum insulin-like growth factor-I, which in turn increased the size and strength of the quadriceps muscle in canines during remobilization.

J. Bone Miner. Res. 1998; 13: 1158-1166, discloses that treatment with the growth hormone secretagogue, MK-0677, increases markers of bone formation and bone resorption in obese young males.

Bone 23 (5) (Supplement), Abstract F235 from ASBMR/IBMS Joint Meeting (November 1998), discloses that the growth hormone secretagogues, GHRP-6 and ipamorelin (IPA), have the capacity to increase bone mass in adult female rats.

R. Bross, M. Javanbakht and S. Bhasin, J.Clin.Endocrinol.Metab. 84:3420-3430 (1999), discusses the potential use of human growth hormone supplementation for the treatment of aging-associated sarcopenia.

### SUMMARY OF THE INVENTION

The present invention provides methods for improving functional health status in a patient in need thereof which comprises administering to the patient a therapeutically effective amount of a growth hormone secretagogue.

More particularly, the present invention provides methods, such as the following: a method wherein the instrumental activities of daily living of the patient are improved; a method wherein the energy level of the patient is improved; a method wherein the mood of the patient is improved; a method wherein the energy level, mood and sleep quality of the patient are improved; a method wherein the cognitive status of the patient is improved; a method wherein the mental acuity of the patient is improved; a method wherein the ability of the patient to perform in the workplace is improved; a method wherein the health-related quality of life of the patient is improved; a method wherein the social isolation of the patient is reduced.

More particularly, the present invention provides such methods wherein the patient is a human. Even more particularly, the present invention provides such methods wherein the human is an elderly or chronically ill individual. Also, the present invention provides such methods wherein the human has age-related decline in physical performance or is growth hormone deficient.

More particularly, the present invention provides such methods wherein the growth hormone secretagogue is an orally active growth hormone secretagogue. Even more particularly, the present invention provides such methods wherein the growth hormone secretagogue is orally administered. Also, the present invention provides such methods wherein the growth hormone secretagogue is a non-peptidyl growth hormone secretagogue.

More preferably, the present invention provides such methods, wherein the growth hormone secretagogue is a compound of Formula I: 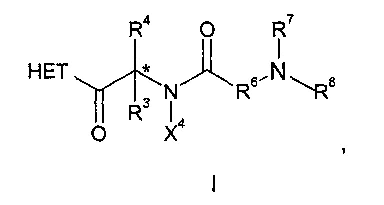 or a stereoisomeric mixture thereof, diastereomerically enriched, diastereomerically pure, enantiomerically enriched or enantiomerically pure isomer thereof, or a prodrug of such compound, mixture or isomer thereof, or a pharmaceutically acceptable salt of the compound, mixture, isomer or prodrug, or a tautomer thereof,
wherein
HET is a heterocyclic moiety selected from the group consisting of 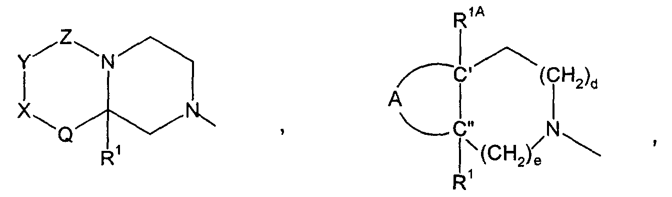 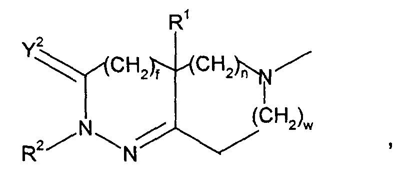 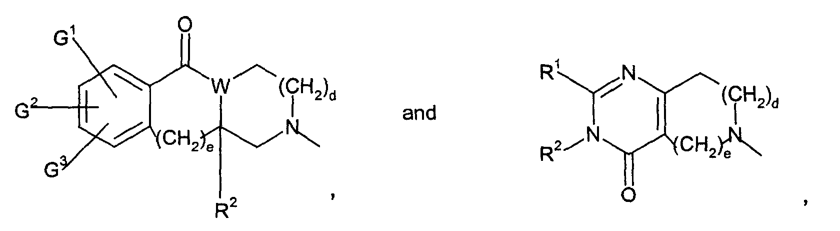
d is 0, 1 or 2;
e is 1 or 2;
f is 0 or 1 ;
n and w are 0, 1 or 2, provided that n and w cannot both be 0 at the same time;
Y² is oxygen or sulfur;
A is a divalent radical, where the left hand side of the radical as shown below is connected to C" and the right hand side of the radical as shown below is connected to C', selected from the group consisting of
   -NR²-C(O)-NR²-, -NR²-S(O)₂-NR²-, -O-C(O)-NR²-, -NR²-C(O)-O-, -C(O)-NR²-C(O)-, -C(O)-NR²-C(R⁹R¹⁰)-, -C(R⁹R¹⁰)-NR²-C(O)-, -C(R⁹R¹⁰)-C(R⁹R¹⁰)-C(R⁹R¹⁰)-, -S(O)₂-C(R⁹R¹⁰)-C(R⁹R¹⁰)-, -C(R⁹R¹⁰)-O-C(O)-, -C(R⁹R¹⁰)-O-C(R⁹R¹⁰)-, -NR²-C(O)-C(R⁹R¹⁰)-, -O-C(O)-C(R⁹R¹⁰)-, -C(R⁹R¹⁰)-C(O)-NR²-, -C(R⁹R¹⁰)-C(O)-O-, -C(O)-NR²-C(R⁹R¹⁰)-C(R⁹R¹⁰)-, -C(O)-O-C(R⁹R¹⁰)-, -C(R⁹R¹⁰)-C(R⁹R¹⁰)-C(R⁹R¹⁰)-C(R⁹R¹⁰)-, -S(O)₂-NR²-C(R⁹R¹⁰)-C(R⁹R¹⁰)-, -C(R⁹R¹⁰)-C(R⁹R¹⁰)-NR²-C(O)-, -C(R⁹R¹⁰)-C(R⁹R¹⁰)-O-C(O)-,-NR²-C(O)-C(R⁹R¹⁰)-C(R⁹R¹⁰)-, -NR²-S(O)₂-C(R⁹R¹⁰)-C(R⁹R¹⁰)-, -O-C(O)-C(R⁹R¹⁰)-C(R⁹R¹⁰)-, -C(R⁹R¹⁰)-C(R⁹R¹⁰)-C(O)-NR²-, -C(R⁹R¹⁰)-C(R⁹R¹⁰)-C(O)-, -C(R⁹R¹⁰)-NR²-C(O)-O-, -C(R⁹R¹⁰)-O-C(O)-NR², -C(R⁹R¹⁰)-NR²-C(O)-NR²-, -NR²-C(O)-O-C(R⁹R¹⁰)-, -NR²-C(O)-NR²-C(R⁹R¹⁰)-, -NR²-S(O)₂-NR²-C(R⁹R¹⁰)-, -O-C(O)-NR²-C(R⁹R¹⁰)-, -C(O)-N=C(R¹¹)-NR²-, -C(O)-NR²-C(R¹¹)=N-, -C(R⁹R¹⁰)-NR¹²-C(R⁹R¹⁰)-, -NR¹²-C(R⁹R¹⁰)-, -NR¹²-C(R⁹R¹⁰)-C(R⁹R¹⁰)-, -C(O)-O-C(R⁹R¹⁰)-C(R⁹R¹⁰)-, -NR²C(R¹¹)=N-C(O)-, -C(R⁹R¹⁰)-C(R⁹R¹⁰)-N(R¹²)-, -C(R⁹R¹⁰)-NR¹²-, -N=C(R¹¹)-NR²-C(O)-, -C(R⁹R¹⁰)-C(R⁹R¹⁰)-NR²-S(O)₂-,-C(R⁹R¹⁰)-C(R⁹R¹⁰)-S(O)₂-NR²-, -C(R⁹R¹⁰)-C(R⁹R¹⁰)-C(O)-O-, -C(R⁹R¹⁰)-S(O)₂-C(R⁹R¹⁰)-, -C(R⁹R¹⁰)-C(R⁹R¹⁰)-S(O)₂-, -O-C(R⁹R¹⁰)-C(R⁹R¹⁰)-, -C(R⁹R¹⁰)-C(R⁹R¹⁰)-O-, -C(R⁹R¹⁰)-C(O)-C(R⁹R¹⁰)-, -C(O)-C(R⁹R¹⁰)-C(R⁹R¹⁰)- and -C(R⁹R¹⁰)-NR²-S(O)₂-NR²-;
Q is a covalent bond or CH₂;
W is CH or N;
X is CR⁹R¹⁰, C=CH₂ or C=O;
Y is CR⁹R¹⁰, O or NR²;
Z is C=O, C=S or S(O)₂;
G¹ is hydrogen, halo, hydroxy, nitro, amino, cyano, phenyl, carboxyl, -CONH₂, -(C₁-C₄)alkyl optionally independently substituted with one or more phenyl, one or more halogens or one or more hydroxy groups, -(C₁-C₄)alkoxy optionally independently substituted with one or more phenyl, one or more halogens or one or more hydroxy groups, -(C₁-C₄)alkylthio, phenoxy, -COO(C₁-C₄)alkyl, N,N-di-(C₁-C₄)alkylamino,-(C₂-C₆)alkenyl optionally independently substituted with one or more phenyl, one or more halogens or one or more hydroxy groups, -(C₂-C₆)alkynyl optionally independently substituted with one or more phenyl, one or more halogens or one or more hydroxy groups, -(C₃-C₆)cycloalkyl optionally independently substituted with one or more (C₁-C₄)alkyl groups, one or more halogens or one or more hydroxy groups, -(C₁-C₄)alkylamino carbonyl or di-(C₁-C₄)alkylamino carbonyl;
G² and G³ are each independently selected from the group consisting of hydrogen, halo, hydroxy, -(C₁-C₄)alkyl optionally independently substituted with one to three halo groups and -(C₁-C₄)alkoxy optionally independently substituted with one to three halo groups;
R¹ is hydrogen, -CN, -(CH₂)_{q}N(X⁶)C(O)X⁶, -(CH₂)_{q}N(X⁶)C(O)(CH₂)ₜ-A¹,
   -(CH₂)_{q}N(X⁶)S(O)₂(CH₂)ₜ-A¹, -(CH₂)_{q}N(X⁶)S(O)₂X⁶, -(CH₂)_{q}N(X⁶)C(O)N(X⁶)(CH₂)ₜ-A¹, -(CH₂)_{q}N(X⁶)C(O)N(X⁶)(X⁶), -(CH₂)_{q}C(O)N(X⁶)(X⁶), -(CH₂)_{q}C(O)N(X⁶)(CH₂)ₜ-A¹, -(CH₂)_{q}C(O)OX⁶, -(CH₂)_{q}C(O)O(CH₂)ₜ-A¹, -(CH₂)_{q}OX⁶, -(CH₂)_{q}OC(O)X⁶, -(CH₂)_{q}OC(O)(CH₂)ₜ-A¹, -(CH₂)_{q}OC(O)N(X⁶)(CH₂)ₜ-A¹, -(CH₂)_{q}OC(O)N(X⁶)(X⁶), -(CH₂)_{q}C(O)X⁶, -(CH₂)_{q}C(O)(CH₂)ₜ-A¹, -(CH₂)_{q}N(X⁶)C(O)OX⁶, -(CH₂)_{q}N(X⁶)S(O)₂N(X⁶)(X⁶), -(CH₂)_{q}S(O)ₘX⁶, -(CH₂)_{q}S(O)ₘ(CH₂)ₜ-A¹, -(C₁-C₁₀)alkyl, -(CH₂)ₜ-A¹, -(CH₂)_{q}-(C₃-C₇)cycloalkyl, -(CH₂)_{q}-Y¹-(C₁-C₆)alkyl, -(CH₂)_{q}-Y¹-(CH₂)ₜ-A¹ or -(CH₂)_{q}-Y¹-(CH₂)ₜ-(C₃-C₇)cycloalkyl;
   where the alkyl and cycloalkyl groups in the definition of R¹ are optionally substituted with (C₁-C₄)alkyl, hydroxy, (C₁-C₄)alkoxy, carboxyl, -CONH₂, -S(O)ₘ(C₁-C₆)alkyl, -CO₂(C₁-C₄)alkyl ester, 1H-tetrazol-5-yl or 1, 2 or 3fluoro groups;
   Y¹ is O, S(O)ₘ, -C(O)NX⁶-, -CH=CH-, -C≡C-, -N(X⁶)C(O)-, -C(O)NX⁶-, -C(O)O-, -OC(O)N(X⁶)- or -OC(O)-;
   q is 0, 1, 2, 3 or 4;
   t is 0, 1, 2 or 3;
   said (CH₂)_{q} group and (CH₂)ₜ group in the definition of R¹ are optionally independently substituted with hydroxy, (C₁-C₄)alkoxy, carboxyl, -CONH₂, -S(O)ₘ(C₁-C₆)alkyl, -CO₂(C₁-C₄)alkyl ester, 1H-tetrazol-5-yl, 1, 2 or 3 fluoro groups or 1 or 2 (C₁-C₄)alkyl groups;
R^{1A} is selected from the group consisting of hydrogen, F, Cl, Br, I, (C₁-C₆)alkyl, phenyl(C₁-C₃)alkyl, pyridyl(C₁-C₃)alkyl, thiazolyl(C₁-C₃)alkyl and thienyl(C₁-C₃)alkyl, provided that R^{1A} is not F, Cl, Br or I when a heteroatom is vicinal to C";
R² is hydrogen, (C₁-C₈)alkyl, -(C₀-C₃)alkyl-(C₃-C₈)cycloalkyl, -(C₁-C₄)alkyl-A¹ or A¹;
   where the alkyl groups and the cycloalkyl groups in the definition of R² are optionally substituted with hydroxy, -C(O)OX⁶, -C(O)N(X⁶)(X⁶), -N(X⁶)(X⁶),-S(O)ₘ(C₁-C₆)alkyl, -C(O)A¹, -C(O)(X⁶), CF₃, CN or 1, 2 or 3 independently selected halo groups;
R³ is selected from the group consisting of A¹, (C₁-C₁₀)alkyl, -(C₁-C₆)alkyl-A¹, -(C₁-C₆)alkyl-(C₃-C₇)cycloalkyl, -(C₁-C₅)alkyl-X¹-(C₁-C₅)alkyl, -(C₁-C₅)alkyl-X¹-(C₀-C₅)alkyl-A¹ and -(C₁-C₅)alkyl-X¹-(C₁-C₅)alkyl-(C₃-C₇)cycloalkyl;
   where the alkyl groups in the definition of R³ are optionally substituted with -S(O)ₘ(C₁-C₆)alkyl, -C(O)OX³, 1, 2, 3, 4 or 5 independently selected halo groups or 1, 2 or 3 independently selected -OX³ groups;
   X¹ is O, S(O)ₘ, -N(X²)C(O)-, -C(O)N(X²)-, -OC(O)-, -C(O)O-, -CX²=CX²-, -N(X²)C(O)O-, -OC(O)N(X²)- or -C≡C-;
R⁴ is hydrogen, (C₁-C₆)alkyl or (C₃-C₇)cycloalkyl, or R⁴ is taken together with R³ and the carbon atom to which they are attached and form (C₅-C₇)cycloalkyl, (C₅-C₇)cycloalkenyl, a partially saturated or fully saturated 4- to 8-membered ring having 1 to 4 heteroatoms independently selected from the group consisting of oxygen, sulfur and nitrogen, or is a bicyclic ring system consisting of a partially saturated or fully saturated 5- or 6-membered ring, fused to a partially saturated, fully unsaturated or fully saturated 5- or 6-membered ring, optionally having 1 to 4 heteroatoms independently selected from the group consisting of nitrogen, sulfur and oxygen;
X⁴ is hydrogen or (C₁-C₆)alkyl or X⁴ is taken together with R⁴ and the nitrogen atom to which X⁴ is attached and the carbon atom to which R⁴ is attached and form a five to seven membered ring;
R⁶ is a bond or is 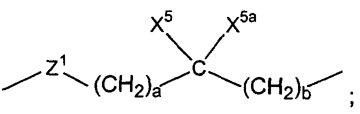
   where a and b are each independently 0, 1, 2 or 3;
   X⁵ and X^{5a} are each independently selected from the group consisting of hydrogen, CF₃, A¹ and optionally substituted (C₁-C₆)alkyl;
      the optionally substituted (C₁-C₆)alkyl in the definition of X⁵ and X^{5a} is optionally substituted with a substituent selected from the group consisting of A¹, OX², -S(O)ₘ(C₁-C₆)alkyl, -C(O)OX², (C₃-C₇)cycloalkyl, -N(X²)(X²) and -C(O)N(X²)(X²);
   or the carbon bearing X⁵ or X^{5a} forms one or two alkylene bridges with the nitrogen atom bearing R⁷ and R⁸ wherein each alkylene bridge contains 1 to 5 carbon atoms, provided that when one alkylene bridge is formed then only one of X⁵ or X^{5a} is on the carbon atom and only one of R⁷ or R⁸ is on the nitrogen atom and further provided that when two alkylene bridges are formed then X⁵ and X^{5a} cannot be on the carbon atom and R⁷ and R⁸ cannot be on the nitrogen atom;
   or X⁵ is taken together with X^{5a} and the carbon atom to which they are attached and form a partially saturated or fully saturated 3- to 7-membered ring, or a partially saturated or fully saturated 4- to 8-membered ring having
   1 to 4 heteroatoms independently selected from the group consisting of oxygen, sulfur and nitrogen;
   or X⁵ is taken together with X^{5a} and the carbon atom to which they are attached and form a bicyclic ring system consisting of a partially saturated or fully saturated 5- or 6-membered ring, optionally having 1 or 2 heteroatoms independently selected from the group consisting of nitrogen, sulfur and oxygen, fused to a partially saturated, fully saturated or fully unsaturated 5-or 6-membered ring, optionally having 1 to 4 heteroatoms independently selected from the group consisting of nitrogen, sulfur and oxygen;
   Z¹ is a bond, O or N-X², provided that when a and b are both 0 then Z¹ is not N-X² or 0;
or R⁶ is -(CR^{a}R^{b})ₐ-E-(CR^{a}R^{b})_{b}-, where the -(CR^{a}R^{b})ₐ- group is attached to the carbonyl carbon of the amide group of the compound of formula I and the -(CR^{a}R^{b})_{b} group is attached to the terminal nitrogen atom of the compound of Formula I;
   E is -O-, -S-, -CH=CH- or an aromatic moiety selected from 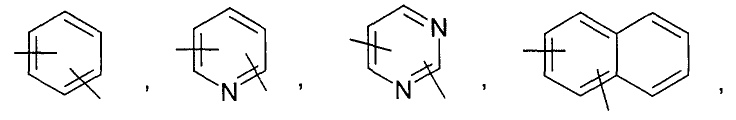 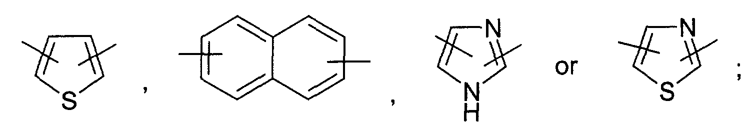 said aromatic moiety in the definition of E optionally substituted with up to three halo, hydroxy, -N(R^{c})(R^{c}), (C₁-C₆)alkyl or (C₁-C₆)alkoxy;
   R^{a} and R^{b} are, for each occurrence, independently hydrogen, (C₁-C₆)alkyl, trifluoromethyl, phenyl or monosubstituted (C₁-C₆)alkyl where the substituents are imidazolyl, naphthyl, phenyl, indolyl, p-hydroxyphenyl, -OR^{c}, S(O)ₘR^{c}, C(O)OR^{c}, (C₃-C₇)cycloalkyl, -N(R^{c})(R^{c}), -C(O)N(R^{c})(R^{c}), or R^{a} or R^{b} may independently be joined to one or both of R⁷ or E (where E is other than O, S or -CH=CH-) to form an alkylene bridge between the terminal nitrogen and the alkyl portion of the R^{a} or R^{b} and the R⁷ or E group, wherein the bridge contains 1 to 8 carbon atoms; or R^{a} and R^{b} may be joined to one another to form a (C₃-C₇)cycloalkyl;
   R^{c}, for each occurrence, is independently hydrogen or (C₁-C₆)alkyl;
   a and b are independently 0, 1, 2 or 3, with the proviso that if E is -O- or -S-, b is other than 0 or 1 and with the further proviso that if E is -CH=CH-, b is other than 0;
R⁷ and R⁸ are each independently hydrogen or optionally substituted (C₁-C₆)alkyl;
   where the optionally substituted (C₁-C₆)alkyl in the definition of R⁷ and R⁸ is optionally independently substituted with A¹, -C(O)O-(C₁-C₆)alkyl, -S(O)ₘ(C₁-C₆)alkyl, 1 to 5 halo groups, 1 to 3 hydroxy groups, 1 to 3 -O-C(O)(C₁-C₁₀)alkyl groups or 1 to 3 (C₁-C₆)alkoxy groups; or
R⁷ and R⁸ can be taken together to form -(CH₂)ᵣ-L-(CH₂)ᵣ-;
   where L is C(X²)(X²), S(O)ₘ or N(X²);
R⁹ and R¹⁰ are each independently selected from the group consisting of hydrogen, fluoro, hydroxy and (C₁-C₅)alkyl optionally independently substituted with 1-5 halo groups;
R¹¹ is selected from the group consisting of (C₁-C₅)alkyl and phenyl optionally substituted with 1-3 substitutents each independently selected from the group consisting of (C₁-C₅)alkyl, halo and (C₁-C₅)alkoxy;
R¹² is selected from the group consisting of (C₁-C₅)alkylsulfonyl, (C₁-C₅)alkanoyl and (C₁-C₅)alkyl where the alkyl portion is optionally independently substituted by 1-5 halo groups;
A¹ for each occurrence is independently selected from the group consisting of (C₅-C₇)cycloalkenyl, phenyl, a partially saturated, fully saturated or fully unsaturated 4- to 8-membered ring optionally having 1 to 4 heteroatoms independently selected from the group consisting of oxygen, sulfur and nitrogen and a bicyclic ring system consisting of a partially saturated, fully unsaturated or fully saturated 5- or 6-membered ring, optionally having 1 to 4 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
   A¹ for each occurrence is independently optionally substituted, on one or optionally both rings if A¹ is a bicyclic ring system, with up to three substituents, each substituent independently selected from the group consisting of F, Cl, Br, I, OCF₃, OCF₂H, CF₃, CH₃, OCH₃, -OX⁶, -C(O)N(X⁶)(X⁶), -C(O)OX⁶, oxo, (C₁-C₆)alkyl, nitro, cyano, benzyl, -S(O)ₘ(C₁-C₆)alkyl, 1H-tetrazol-5-yl, phenyl, phenoxy, phenylalkyloxy, halophenyl, methylenedioxy, -N(X⁶)(X⁶), -N(X⁶)C(O)(X⁶), -S(O)₂N(X⁶)(X⁶), -N(X⁶)S(O)₂-phenyl, -N(X⁶)S(O)₂X⁶, -CONX¹¹X¹², -S(O)₂NX¹¹X¹², -NX⁶S(O)₂X¹², -NX⁶CONX¹¹X¹², -NX⁶S(O)₂NX¹¹X¹², -NX⁶C(O)X¹², imidazolyl, thiazolyl and tetrazolyl, provided that if A¹ is optionally substituted with methylenedioxy then it can only be substituted with one methylenedioxy;
      where X¹¹ is hydrogen or optionally substituted (C₁-C₆)alkyl;
         the optionally substituted (C₁-C₆)alkyl defined for X¹¹ is optionally independently substituted with phenyl, phenoxy, (C₁-C₆)alkoxycarbonyl, -S(O)ₘ(C₁-C₆)alkyl, 1 to 5 halo groups, 1 to 3 hydroxy groups, 1 to 3 (C₁-C₁₀)alkanoyloxy groups or 1 to 3 (C₁-C₆)alkoxy groups;
      X¹² is hydrogen, (C₁-C₆)alkyl, phenyl, thiazolyl, imidazolyl, furyl or thienyl, provided that when X¹² is not hydrogen, the X¹² group is optionally substituted with one to three substituents independently selected from the group consisting of Cl, F, CH₃, OCH₃, OCF₃ and CF₃;
      or X¹¹ and X¹² are taken together to form -(CH₂)ᵣ-L¹-(CH₂)ᵣ-;
L¹ is C(X²)(X²), O, S(O)ₘ or N(X²);
r for each occurrence is independently 1, 2 or 3;
X² for each occurrence is independently hydrogen, optionally substituted (C₁-C₆)alkyl or optionally substituted (C₃-C₇)cycloalkyl, where the optionally substituted (C₁-C₆)alkyl and optionally substituted (C₃-C₇)cycloalkyl in the definition of X² are optionally independently substituted with -S(O)ₘ(C₁-C₆)alkyl, -C(O)OX³, 1 to 5 halo groups or 1-3 OX³ groups;
X³ for each occurrence is independently hydrogen or (C₁-C₆)alkyl;
X⁶ for each occurrence is independently hydrogen, optionally substituted (C₁-C₆)alkyl, (C₂-C₆)halogenated alkyl, optionally substituted (C₃-C₇)cycloalkyl, (C₃-C₇)-halogenated cycloalkyl, where optionally substituted (C₁-C₆)alkyl and optionally substituted (C₃-C₇)cycloalkyl in the definition of X⁶ is optionally independently mono- or di-substituted with (C₁-C₄)alkyl, hydroxy, (C₁-C₄)alkoxy, carboxyl, CONH₂, -S(O)ₘ(C₁-C₆)alkyl, carboxylate (C₁-C₄)alkyl ester or 1H-tetrazol-5-yl; or
when there are two X⁶ groups on one atom and both X⁶ are independently (C₁-C₆)alkyl, the two (C₁-C₆)alkyl groups may be optionally joined and, together with the atom to which the two X⁶ groups are attached, form a 4- to 9- membered ring optionally having oxygen, sulfur or NX⁷ as a ring member;
   X⁷ is hydrogen or (C₁-C₆)alkyl optionally substituted with hydroxy;
m for each occurrence is independently 0, 1 or 2;
with the provisos that:
1) X⁶ and X¹² cannot be hydrogen when attached to C(O) or S(O)₂ in the form C(O)X⁶, C(O)X¹², S(O)₂X⁶ or S(O)₂X¹²; and
2) when R⁶ is a bond then L is N(X²) and each r in the definition -(CH₂)ᵣ-L-(CH₂)ᵣ- is independently 2 or 3.

More preferably, the present invention provides such methods wherein the growth hormone secretagogue is a compound having the structural formula below, which is designated herein as Formula I-A 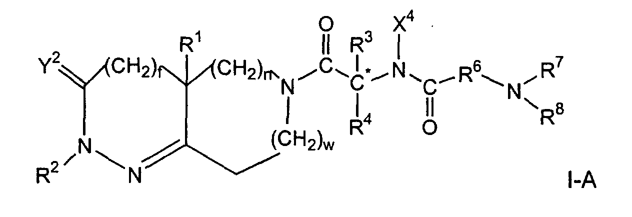 a racemic-diastereomeric mixture or optical isomer of said compound or a pharmaceutically-acceptable salt or a prodrug thereof, or a tautomer thereof,
wherein
f is 0;
n is 0 and w is 2, or n is 1 and w is 1, or n is 2 and w is 0;
Y is oxygen or sulfur;
R¹ is hydrogen, -CN, -(CH₂)_{q}N(X⁶)C(O)X⁶, -(CH₂)_{q}N(X⁶)C(O)(CH₂)ₜ-A¹, -(CH₂)_{q}N(X⁶)SO₂(CH₂)ₜ-A¹, -(CH₂)_{q}N(X⁶)SO₂X⁶, -(CH₂)_{q}N(X⁶)C(O)N(X⁶)(CH₂)ₜ-Al, -(CH₂)_{q}N(X⁶)C(O)N(X⁶)(X⁶), -(CH₂)_{q}C(O)N(X⁶)(X⁶), -(CH₂)_{q}C(O)N(X⁶)(CH₂)ₜ-A¹, -(CH₂)_{q}C(O)OX⁶, -(CH₂)_{q}C(O)O(CH₂)ₜ-A¹, -(CH₂)_{q}OX⁶, -(CH₂)_{q}OC(O)X⁶, -(CH₂)_{q}OC(O)(CH₂)ₜ-A¹, -(CH₂)_{q}OC(O)N(X⁶)(CH₂)ₜ-A¹, -(CH₂)_{q}OC(O)N(X⁶)(X⁶), -(CH₂)_{q}C(O)X⁶, -(CH₂)_{q}C(O)(CH₂)ₜ-A¹, -(CH₂)_{q}N(X⁶)C(O)OX⁶, -(CH₂)_{q}N(X⁶)SO₂N(X⁶)(X⁶), -(CH₂)_{q}S(O)ₘX⁶, -(CH₂)_{q}S(O)ₘ(CH₂)ₜ-A¹, -(C₁-C₁₀)alkyl, -(CH₂)ₜ-A¹, -(CH₂)_{q}-(C₃-C₇)cycloalkyl, -(CH₂)_{q}-Y¹-(C₁-C₆)alkyl, -(CH₂)_{q}-Y¹-(CH₂)ₜ-A¹ or -(CH₂)_{q}-Y¹-(CH₂)ₜ-(C₃-C₇)cycloalkyl;
   where the alkyl and cycloalkyl groups in the definition of R¹ are optionally substituted with (C₁-C₄)alkyl, hydroxyl, (C₁-C₄)alkoxy, carboxyl, -CONH₂, -S(O)ₘ(C₁-C₆)alkyl, -CO₂(C₁-C₄)alkyl ester, 1 H-tetrazol-5-yl or 1, 2 or 3 fluoro;
   Y¹ is O, S(O)ₘ, -C(O)NX⁶-, -CH=CH-, -C≡C-, -N(X⁶)C(O)-, -C(O)O-, -OC(O)N(X⁶)- or -OC(O)-;
   q is 0, 1, 2, 3 or 4;
   t is 0, 1, 2 or 3;
   said (CH₂)_{q} group and (CH₂)ₜ group may each be optionally substituted with hydroxyl, (C₁-C₄)alkoxy, carboxyl, -CONH₂, -S(O)ₘ(C₁-C₆)alkyl,
   -CO₂(C₁-C₄)alkyl ester, 1H-tetrazol-5-yl, 1, 2 or 3 fluoro, or 1 or 2 (C₁-C₄)alkyl;
R² is hydrogen, (C₁-C₈)alkyl, -(C₀-C₃)alkyl-(C₃-C₈)cycloalkyl, -(C₁-C₄)alkyl-A¹ or A¹;
   where the alkyl groups and the cycloalkyl groups in the definition of R² are optionally substituted with hydroxyl, -C(O)OX⁶, -C(O)N(X⁶)(X⁶),
   -N(X⁶)(X⁶), -S(O)ₘ(C₁-C₆)alkyl, -C(O)A¹, -C(O)(X⁶), CF₃, CN or 1, 2 or 3 halogen;
R³ is A¹, (C₁-C₁₀)alkyl, -(C₁-C₆)alkyl-A¹, -(C₁-C₆)alkyl-(C₃-C₇)cycloalkyl, -(C₁-C₅)alkyl-X¹-(C₁-C₅)alkyl, -(C₁-C₅)alkyl-X¹-(C₀-C₅)alkyl-A¹ or -(C₁-C₅)alkyl-X¹-(C₁-C₅)alkyl-(C₃-C₇)cycloalkyl;
   where the alkyl groups in the definition of R³ are optionally substituted with, -S(O)ₘ(C₁-C₆)alkyl, -C(O)OX³, 1, 2, 3, 4 or 5 halogens, or 1, 2 or 3 OX³;
   X¹ is O, S(O)ₘ, -N(X²)C(O)-, -C(O)N(X²)-, -OC(O)-, -C(O)O-, -CX²=CX²-, -N(X²)C(O)O-, -OC(O)N(X²)- or -C≡C-;
R⁴ is hydrogen, (C₁-C₆)alkyl or (C₃-C₇)cycloalkyl;
X⁴ is hydrogen or (C₁-C₆)alkyl or X⁴ is taken together with R⁴ and the nitrogen atom to which X⁴ is attached and the carbon atom to which R⁴ is attached and form a five to seven membered ring;
R⁶ is a bond or is 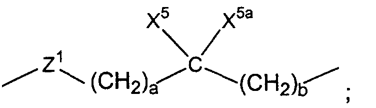 where a and b are independently 0, 1, 2 or 3;
   X⁵ and X^{5a} are each independently selected from the group consisting of hydrogen, trifluoromethyl, A¹ and optionally substituted (C₁-C₆)alkyl;
      the optionally substituted (C₁-C₆)alkyl in the definition of X⁵ and X^{5a} is optionally substituted with a substituent selected from the group consisting of A¹, OX², -S(O)ₘ(C₁-C₆)alkyl, -C(O)OX², (C₃-C₇)cycloalkyl, -N(X²)(X²) and -C(O)N(X²)(X²);
R⁷ and R⁸ are independently hydrogen or optionally substituted (C₁-C₆)alkyl;
   where the optionally substituted (C₁-C₆)alkyl in the definition of R⁷ and R⁸ is optionally independently substituted with A¹, -C(O)O-(C₁-C₆)alkyl, -S(O)ₘ(C₁-C₆)alkyl, 1 to 5 halogens, 1 to 3 hydroxy, 1 to 3 -O-C(O)(C₁-C₁₀)alkyl or 1 to 3 (C₁-C₆)alkoxy; or
R⁷ and R⁸ can be taken together to form -(CH₂)ᵣ-L-(CH₂)ᵣ-;
   where L is C(X²)(X²), S(O)ₘ or N(X²);
A¹ in the definition of R¹ is a partially saturated, fully saturated or fully unsaturated 4- to 8-membered ring optionally having 1 to 4 heteroatoms independently selected from the group consisting of oxygen, sulfur and nitrogen, a bicyclic ring system consisting of a partially saturated, fully unsaturated or fully saturated 5- or 6-membered ring, having 1 to 4 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
A¹ in the definition of R², R³, R⁶, R⁷ and R⁸ is independently (C₅-C₇)cycloalkenyl, phenyl or a partially saturated, fully saturated or fully unsaturated 4- to 8-membered ring optionally having 1 to 4 heteroatoms independently selected from the group consisting of oxygen, sulfur and nitrogen, a bicyclic ring system consisting of a partially saturated, fully unsaturated or fully saturated 5- or 6- membered ring, optionally having 1 to 4 heteroatoms independently selected from the group consisting of nitrogen, sulfur and oxygen, fused to a partially saturated, fully saturated or fully unsaturated 5- or 6- membered ring, optionally having 1 to 4 heteroatoms independently selected from the group consisting of nitrogen, sulfur and oxygen;
   A¹ for each occurrence is independently optionally substituted, in one or optionally both rings if A¹ is a bicyclic ring system, with up to three substituents, each substituent independently selected from the group consisting of F, Cl, Br, I, OCF₃, OCF₂H, CF₃, CH₃, OCH₃, -OX⁶, -C(O)N(X⁶)(X⁶), -C(O)OX⁶, oxo, (C₁-C₆)alkyl, nitro, cyano, benzyl, -S(O)ₘ(C₁-C₆)alkyl, 1H-tetrazol-5-yl, phenyl, phenoxy, phenylalkyloxy, halophenyl, methylenedioxy, -N(X⁶)(X⁶), -N(X⁶)C(O)(X⁶), -SO₂N(X⁶)(X⁶), -N(X⁶)SO₂-phenyl, -N(X⁶)SO₂X⁶, -CONX¹¹X¹², -SO₂NX¹¹X¹², -NX⁶SO₂X¹², -NX⁶CONX¹¹X¹², -NX⁶SO₂NX¹¹X¹², -NX⁶C(O)X¹², imidazolyl, thiazolyl or tetrazolyl, provided that if A¹ is optionally substituted with methylenedioxy then it can only be substituted with one methylenedioxy;
      where X¹¹ is hydrogen or optionally substituted (C₁-C₆)alkyl;
         the optionally substituted (C₁-C₆)alkyl defined for X¹¹ is optionally independently substituted with phenyl, phenoxy, (C₁-C₆)alkoxycarbonyl, -S(O)ₘ(C₁-C₆)alkyl 1 to 5 halogens, 1 to 3 hydroxy, 1 to 3 (C₁-C₁₀)alkanoyloxy or 1 to 3 (C₁-C₆)alkoxy;
      X¹² is hydrogen, (C₁-C₆)alkyl, phenyl, thiazolyl, imidazolyl, furyl or thienyl, provided that when X¹² is not hydrogen, X¹² is optionally substituted with one to three substituents independently selected from the group consisting of Cl, F, CH₃, OCH₃, OCF₃ and CF₃;
      or X¹¹ and X¹² are taken together to form -(CH₂)ᵣ-L¹-(CH₂)ᵣ-;
         where L¹ is C(X²)(X²), O, S(O)ₘ or N(X²);
   r for each occurrence is independently 1, 2 or 3;
   X² for each occurrence is independently hydrogen, optionally substituted (C₁-C₆)alkyl, or optionally substituted (C₃-C₇)cycloalkyl, where the optionally substituted (C₁-C₆)alkyl and optionally substituted (C₃-C₇)cycloalkyl in the definition of X² are optionally independently substituted with -S(O)ₘ(C₁-C₆)alkyl, -C(O)OX³, 1 to 5 halogens or 1-3 OX³;
   X³ for each occurrence is independently hydrogen or (C₁-C₆)alkyl;
   X⁶ is independently hydrogen, optionally substituted (C₁-C₆)alkyl, (C₂-C₆)halogenated alkyl, optionally substituted (C₃-C₇)cycloalkyl, (C₃-C₇)-halogenatedcycloalkyl, where optionally substituted (C₁-C₆)alkyl and optionally substituted (C₃-C₇)cycloalkyl in the definition of X⁶ is optionally independently substituted by 1 or 2 (C₁-C₄)alkyl, hydroxyl, (C₁-C₄)alkoxy, carboxyl, CONH₂,-S(O)ₘ(C₁-C₆)alkyl, carboxylate (C₁-C₄)alkyl ester, or 1H-tetrazol-5-yl; or
   when there are two X⁶ groups on one atom and both X⁶ are independently (C₁-C₆)alkyl, the two (C₁-C₆)alkyl groups may be optionally joined and, together with the atom to which the two X⁶ groups are attached, form a 4- to 9- membered ring optionally having oxygen, sulfur or NX⁷;
   X⁷ is hydrogen or (C₁-C₆)alkyl optionally substituted with hydroxyl; and
   m for each occurrence is independently 0, 1 or 2;
   with the proviso that:
   X⁶ and X¹² cannot be hydrogen when it is attached to C(O) or SO₂ in the form C(O)X⁶, C(O)X¹², SO₂X⁶ or SO₂X¹²; and
   when R⁶ is a bond then L is N(X²) and each r in the definition -(CH₂)ᵣ-L-(CH₂)ᵣ- is independently 2 or 3.

More preferably, the present invention provides such methods wherein the growth hormone secretagogue is 2-amino-N-(2-(3a-(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl)-isobutyramide, a prodrug thereof or a pharmaceutically acceptable salt of the growth hormone secretagogue or the prodrug. Even more preferably, the present invention provides such methods wherein the growth hormone secretagogue is 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide, L-tartrate.

Also, more preferably, the present invention provides such methods wherein the growth hormone secretagogue is 2-amino-N-(1-(R)-(2,4-difluorobenzyloxymethyl)-2-oxo-2-(3-oxo-3a-(R)-pyridin-2-ylmethyl-2-(2,2,2-trifluoro-ethyl)-2,3,3a,4,6,7-hexahydro-pyrazolo-[4,3-c]pyridin-5-yl)-ethyl)-2-methyl-propionamide, a prodrug thereof or a pharmaceutically acceptable salt of the growth hormone secretagogue or the prodrug. Even more preferably, the present invention provides such methods wherein the growth hormone secretagogue is the (L)-(+)-tartaric acid salt of 2-amino-N-(1-(R)-(2,4-difluoro-benzyloxymethyl)-2-oxo-2-(3-oxo-3a-(R)-pyridin-2-ylmethyl-2-(2,2,2-trifluoro-ethyl)-2,3,3a,4,6,7-hexahydro-pyrazolo-[4,3-c]pyridin-5-yl)-ethyl)-2-methyl-propionamide.

Also, more preferably, the present invention provides such methods wherein the growth hormone secretagogue is 2-amino-N-(1-(R)-benzyloxymethyl-2-(1,3-dioxo-8a(S)-pyridin-2-ylmethyl-2-(2,2,2-trifluoro-ethyl)-hexahydro-imidazo[1,5-a]pyrazin-7-yl)-2-oxo-ethyl)-2-methyl-propionamide, a prodrug thereof or a pharmaceutically acceptable salt of the growth hormone secretagogue or the prodrug. Even more preferably, the present invention provides such methods wherein the growth hormone secretagogue is the (L)-(+)-tartaric acid salt of 2-amino-N-(1-(R)-benzyloxymethyl-2-(1,3-dioxo-8a(S)-pyridin-2-ylmethyl-2-(2,2,2-trifluoro-ethyl)-hexahydro-imidazo[1,5-a]pyrazin-7-yl)-2-oxo-ethyl)-2-methylpropionamide.

In addition, the present invention provides methods, as described above, which further comprise administering recombinant growth hormone or a growth hormone secretagogue selected from the group consisting of GHRP-6, GHRP-1, GHRP-2, hexarelin, growth hormone releasing factor, an analog of growth hormone releasing factor, IGF-I and IGF-II.

In addition, the present invention provides methods, as described above, which further comprise administering an antidepressant, a prodrug thereof or a pharmaceutically acceptable salt of said antidepressant or said prodrug. More particularly, the present invention provides such methods wherein said antidepressant is a norepinephrine reuptake inhibitor (NERI), selective serotonin reuptake inhibitor (SSRI), monoamine oxidase inhibitor (MAO), combined NERI/SSRI, or an atypical antidepressant, a prodrug of said antidepressant or a pharmaceutically acceptable salt of said antidepressant or said prodrug. More particularly, the present invention provides such methods wherein said antidepressant is a selective serotonin reuptake inhibitor (SSRI), a prodrug thereof or a pharmaceutically acceptable salt of said SSRI or said prodrug. More particularly, the present invention provides such methods wherein said SSRI is citalopram, femoxetine, fluoxetine, fluvoxamine, indalpine, indeloxazine, milnacipran, paroxetine, sertraline, sibutramine or zimeldine, a prodrug of said SSRI or a pharmaceutically acceptable salt of said SSRI or said prodrug. Even more particularly, the present invention provides such methods wherein said SSRI is sertraline, a prodrug thereof or a pharmaceutically acceptable salt of sertraline or said prodrug. Even more specifically, the present invention provides such methods wherein said SSRI is sertraline hydrochloride.

In addition, the present invention provides methods, as described above, which further comprise administering an antipsychotic agent, a prodrug thereof or a pharmaceutically acceptable salt of said antipsychotic agent or said prodrug. Even more particularly, the present invention provides such methods wherein the antipsychotic agent is chlorpromazine, haloperidol, clozapine, loxapine, molindone hydrochloride, thiothixene, olanzapine, ziprasidone hydrochloride, prochlorperazine, perphenazine, trifluoperazine hydrochloride or risperidone.

In addition, the present invention provides methods, as described above, which further comprises administering an antianxiety agent, a prodrug thereof or a pharmaceutically acceptable salt of said antianxiety agent or said prodrug. Even more particularly, the present invention provides such methods wherein the antianxiety agent is alprazolam, clonazepam, lorazepam, oxazepam, chlordiazepoxide hydrochloride, diazepam, buspirone hydrochloride, doxepin hydrochloride, hydroxyzine pamoate or clonazepam.

In addition, the present invention provides methods, as described above, which further comprise administering a naturaceutic selected from ginko biloba, St. John's Wart, valerian and melatonin.

### DETAILED DESCRIPTION OF THE INVENTION

The present invention is directed to the use of a compound, which has the ability to stimulate or amplify the release of endogenous growth hormone, for improving functional health status in a patient in need thereof. In particular, the present invention provides a method for improving functional health status in a patient in need thereof comprising the administration to the patient of a growth hormone secretagogue, preferably a compound of Formula I below.

In the present invention, it is preferred that the patient is a human. Although the present invention is applicable to both old and young people, it may find greater application in elderly people or in chronically ill people. It may also find application in people who have age-related decline in physical performance and in people who are growth hormone deficient. It is also preferred that the patient is a companion animal such as a cat or dog, with an elderly companion animal being particularly preferred.

By the term "growth hormone secretagogue" is meant any exogenously administered compound or agent that directly or indirectly stimulates or increases the endogenous release of growth hormone, growth hormone-releasing hormone or somatostatin in an animal, in particular, a human. This term shall at all times be understood to include all active forms of such secretagogues, including, for example, the free form thereof, e.g., the free acid or base form, and also, all prodrugs, polymorphs, hydrates, solvates, stereoisomers, e.g., diastereomers and enantiomers, and the like, and all pharmaceutically acceptable salts as described above, unless specifically stated otherwise. It will also be appreciated that suitable active metabolites of secretagogues within the scope of the present invention, in any suitable form, are also included herein.

The growth hormone secretagogue may be peptidyl or non-peptidyl in nature; however, the use of an orally active growth hormone secretagogue is preferred. In addition, it is preferred that the growth hormone secretagogue induce or amplify a pulsatile release of endogenous growth hormone.

The expression "prodrug" refers to compounds that are drug precursors which, following administration, release the drug *in vivo* via some chemical or physiological process (e.g., a prodrug on being brought to the physiological pH is converted to the desired drug form). For example, a prodrug of the compound of Formula I may be used in the present invention. Exemplary prodrugs are disclosed in the art, particularly in the references cited herein and incorporated herein by reference.

Representative growth hormone secretagogues are disclosed in the following International Patent Applications (listed by Publication Nos.), issued U.S. patents and published European patent applications, which are incorporated herein by reference: WO 98/46569, WO 98/51687, WO 98/58947, WO 98/58949, WO 98/58950, WO 99/08697, WO 99/09991, WO 95/13069, U.S. 5,492,916, U.S. 5,494,919, WO 95/14666, WO 94/19367, WO 94/13696, WO 94/11012, U.S. 5,726,319, WO 95/11029, WO 95/17422, WO 95/17423, WO 95/34311, WO 96/02530, WO 96/22996, WO 96/22997, WO 96/24580, WO 96/24587, U.S. 5,559,128, WO 96/32943, WO 96/33189, WO 96/15148, WO 96/38471, WO 96/35713, WO 97/00894, WO 97/07117, WO 97/06803, WO 97/11697, WO 97/15573, WO 97/22367, WO 97/23508, WO 97/22620, WO 97/22004, WO 97/21730, WO 97/24369, U.S. 5,663,171, WO 97/34604, WO 97/36873, WO 97/40071, WO 97/40023, WO 97/41878, WO97/41879, WO 97/46252, WO 97/44042, WO 97/38709, WO 98/03473, WO 97/43278, U.S. 5,721,251, U.S. 5,721,250, WO 98/10653, U.S. 5,919,777, U.S. 5,830,433 and EP 0995748.

A representative first group of growth hormone secretagogues is set forth in International Patent Application, Publication No. WO 97/24369, as compounds having the structural formula below, which is designated herein as Formula II: 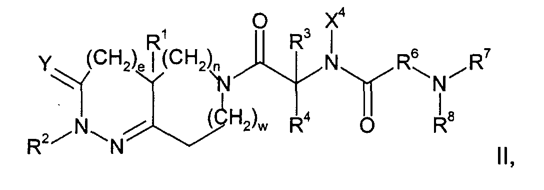 wherein the various substituents are as defined in WO 97/24369. Said compounds are prepared as disclosed therein.

2-Amino-N-(2-(3a-(R)-benzyl-2-methyl-3-oxo-2,3,3a,4,6,7-hexahydropyrazolo-[4,3-c]pyridin-5-yl)-1-(R)-benzyloxymethyl-2-oxo-ethyl)-isobutyramide, having the following structure: 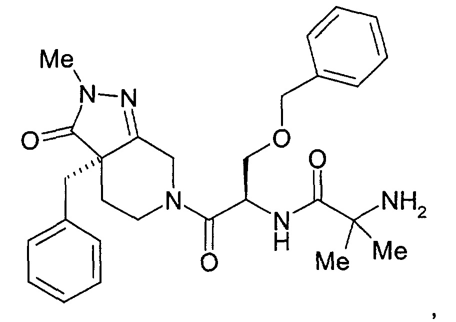 and 2-amino-N-(1-(R)-(2,4-difluoro-benzyloxymethyl)-2-oxo-2-(3-oxo-3a-(R)-pyridin-2-ylmethyl)-2-(2,2,2-trifluoro-ethyl)-2,3,3a,4,6,7-hexahydro-pyrazolo-[4,3-c]pyridin-5-yl)-ethyl)-2-methyl-propionamide, having the following structure: 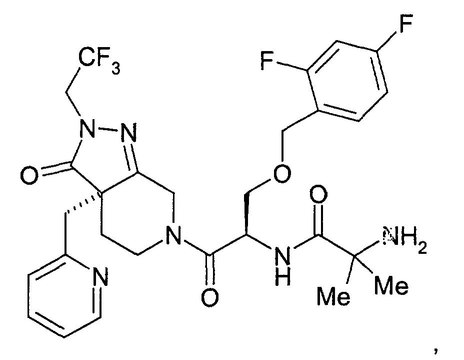 and the pharmaceutically acceptable salts thereof, are within the scope of the disclosure of International Patent Application, Publication Number WO 97/24369.

A representative second group of growth hormone secretagogues is set forth in International Patent Application, Publication No. WO 98/58947, as compounds having the structural formula below, which is designated herein as Formula III: 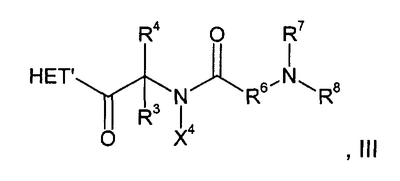 wherein the various substituents are as defined in WO 98/58947. Said compounds are prepared as disclosed therein.

A preferred compound within this second group which may be employed in the present invention is identified as having the following name and structure: 2-amino-N-(1 (R)-benzyloxymethyl-2-(1,3-dioxo-8a(S)-pyridin-2-ylmethyl-2-(2,2,2-trifluoro-ethyl)-hexahydro-imidazo[1,5-a]pyrazin-7-yl)-2-oxo-ethyl)-2-methylpropionamide, 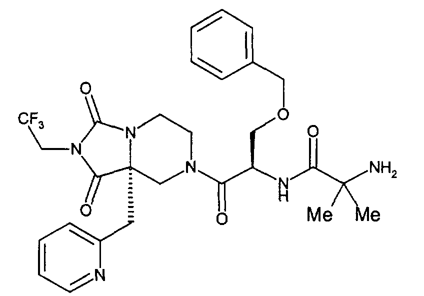 This compound and pharmaceutically acceptable salts thereof are within the scope of the disclosure of International Patent Application, Publication No. WO 98/58947, and may be prepared as described in Examples Five and Six therein.

A representative third group of growth hormone secretagogues is set forth in published European patent application 0995748, which discloses certain dipeptide growth hormone secretagogues of the structural formula above, which is designated herein as Formula III, wherein the variables are as defined in EP 0995748, and their use for the treatment or prevention of musculoskeletal fraility including osteoporosis.

A representative fourth group of growth hormone secretagogues is set forth in U.S. Patent No. 5,206,235, as having the following structure: 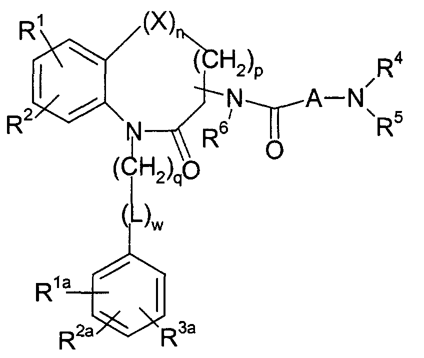 wherein the various substituents are as defined in U.S. Patent No. 5,206,235. Said compounds are prepared as disclosed therein.

Preferred compounds within this fourth group are identified as having the following structures: 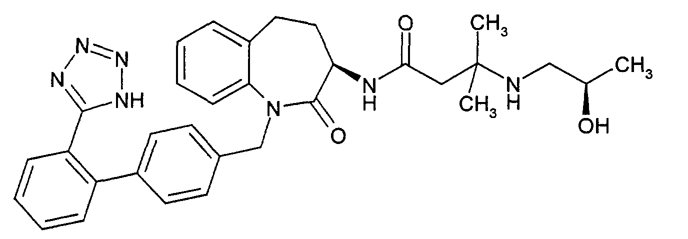 and

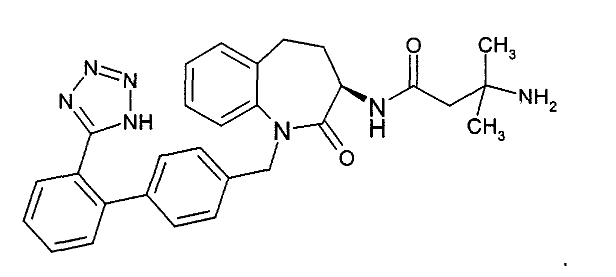

A representative fifth group of growth hormone secretagogues is set forth in U.S. Patent No. 5,283,241, as having the following structural formula: 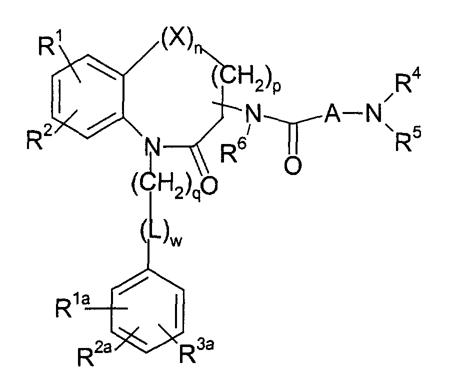 wherein the various substituents are as defined in U.S. Patent 5,283,241. Said compounds are prepared as disclosed therein.

A representative sixth group of growth hormone secretagogues is disclosed in International Patent Application, Publication No. WO 97/41879, as compounds having the following structural formulas: 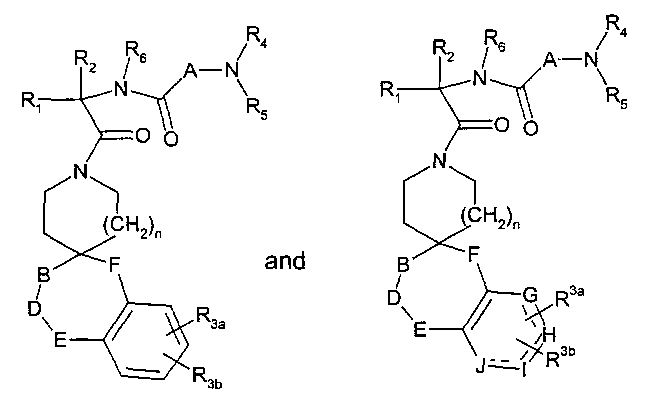 wherein the various substituents are as defined in WO97/41879. Said compounds are prepared as disclosed therein.

Preferred compounds within this sixth group which may be employed in the present invention are identified as having the following structure: 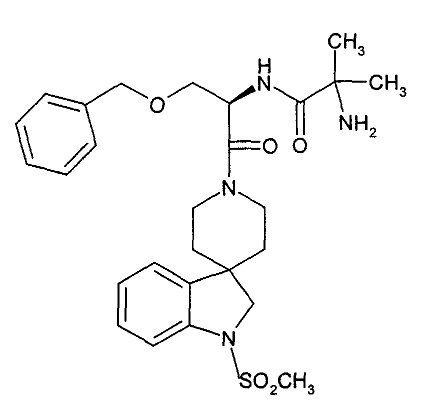 and pharmaceutically acceptable salts thereof, in particular, the methanesulfonate salt.

A representative seventh group of growth hormone secretagogues is disclosed in U.S. Patent No. 5,492,916, as being compounds of the following structural formula: 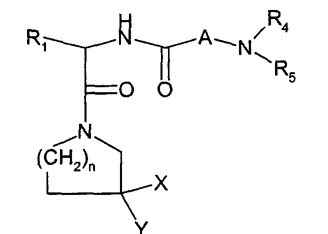 wherein the various substituents are as defined in U.S. Patent No. 5,492,916. Said compounds are prepared as disclosed therein.

All of the compounds identified above may be prepared by procedures disclosed in the cited publications. Full descriptions of the preparation of the growth hormone secretagogues which may be employed in the present invention may be found in the art, particularly in the references cited herein, which are incorporated by reference herein.

The compounds identified above and used in the methods of the present invention may have one or more asymmetric centers. The compounds of Formula I used in the methods of the present invention all have at least one asymmetric center as noted, e.g., by the asterisk in the structural Formula I-B below. Additional asymmetric centers may be present in the compounds of Formula I depending upon the nature of the various substituents on the molecule. Each such asymmetric center will produce two optical isomers and it is intended that all such optical isomers, as separated, pure or partially purified optical isomers, racemic mixtures or diastereomeric mixtures thereof, be included within the scope of the methods of the present invention. In the case of the asymmetric center represented by the asterisk, it has been found that the absolute stereochemistry of the more active and thus more preferred isomer is shown in Formula I-B below: 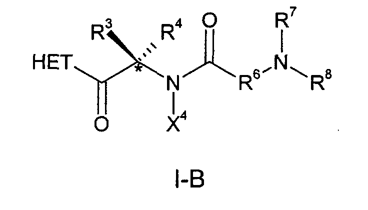 With the R⁴ substituent as hydrogen, the spatial configuration of the asymmetric center corresponds to that in a D-amino acid. In most cases this is also designated an R-configuration although this will vary according to the values of R³ and R⁴ used in making R- or S-stereochemical assignments.

Certain compounds within the scope of the present invention may have the potential to exist in different tautomeric forms. All tautomers of a compound of the present invention are within the scope of the present invention. Also, for example, all keto-enol or imine-enamine forms of the compounds are included in the present invention. Those skilled in the art will recognize that the compound names contained herein may be based on a particular tautomer of a compound. While the name for only a particular tautomer may be used, it is intended that all tautomers are encompassed by the name of the particular tautomer and ali tautomers are considered part of the present invention.

A compound within the scope of the present invention may exist in unsolvated as well as solvated forms with pharmaceutically acceptable solvents such as water, ethanol, and the like. A solvate wherein the solvent is water forms a hydrate or hydrated ions. The present invention contemplates and encompasses both the solvated and unsolvated forms of the compounds within its scope.

Also included within the scope of the present invention are isotopically-labelled compounds, which are identical to those recited herein, but for the fact that one or more atoms are replaced by an atom having an atomic mass or mass number different from the atomic mass or mass number usually found in nature. Examples of isotopes that can be incorporated into compounds of the present invention include isotopes of hydrogen, carbon, nitrogen, oxygen, phosphorous, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively. Compounds of the present invention, prodrugs thereof, and pharmaceutically acceptable salts of said compounds or of said prodrugs which contain the aforementioned isotopes and/or other isotopes of other atoms are within the scope of this invention. Certain isotopically-labelled compounds of the present invention, for example those into which radioactive isotopes such as ³H and ¹⁴C are incorporated, are useful in compound and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, may afford certain therapeutic advantages resulting from greater metabolic stability, for example increased *in vivo* half-life or reduced dosage requirements and, hence, may be preferred in some circumstances. Isotopically labelled compounds of the present invention and prodrugs thereof can generally be prepared by carrying out the procedures disclosed in the references cited herein as well as others known in the art, by substituting a readily available isotopically labelled reagent for a non-isotopically labelled reagent.

Full descriptions of the preparation of the growth hormone secretagogues employed in the present invention may be found, for example, in the following International Patent Applications (listed by Publication Nos.), issued U.S. patents and published European patent applications, which are incorporated herein by reference: WO 98/46569, WO 98/51687, WO 98/58947, WO 98/58949, WO 98/58950, WO 99/08697, WO 99/09991, WO 95/13069, U.S. 5,492,916, U.S. 5,494,919, WO 95/14666, WO 94/19367, WO 94/13696, WO 94/11012, U.S. 5,726,319, WO 95/11029, WO 95/17422, WO 95/17423, WO 95/34311, WO 96/02530, WO 96/22996, WO 96/22997, WO 96/24580, WO 96/24587, U.S. 5,559,128, WO 96/32943, WO 96/33189, WO 96/15148, WO 96/38471, WO 96/35713, WO 97/00894, WO 97/07117, WO 97/06803, WO 97/11697, WO 97/15573, WO 97/22367, WO 97/23508, WO 97/22620, WO 97/22004, WO 97/21730, WO 97/24369, U.S. 5,663,171, WO 97/34604, WO 97/36873, WO 97/40071, WO 97/40023, WO 97/41878, WO97/41879, WO 97/46252, WO 97/44042, WO 97/38709, WO 98/03473, WO 97/43278, U.S. 5,721,251, U.S. 5,721,250, WO 98/10653, U.S. 5,919,777, U.S. 5,830,433 and EP 0995748.

A growth hormone secretagogue is a compound that, when administered to a patient, increases the production and/or secretion of growth hormone when compared with baseline plasma concentrations of growth hormone in a normal healthy individual. Thus, to identify a growth hormone secretagogue, one need simply measure the baseline plasma concentrations of growth hormone over a time period, typically one day, and compare the plasma concentrations of growth hormone after administration of a growth hormone secretagogue with the baseline concentration over the time period. Various examples of growth hormone secretagogues are disclosed herein. It is contemplated that any growth hormone secretagogue can be used in the present administration methods.

The identification of a compound as a "growth hormone secretagogue" which is able to directly or indirectly stimulate or increase the endogenous release of growth hormone in an animal may be readily determined without undue experimentation by methodology well known in the art, such as the assay described by Smith et al., Science, 260, 1640-1643 (1993) (see text of Figure 2 therein). In a typical experiment, pituitary glands are aseptically removed from 150-200 g Wistar male rats and cultures of pituitary cells are prepared according to Cheng et al., Endocrinol., 124, 2791-2798 (1989). The cells are treated with the subject compound and assayed for growth hormone secreting activity, as described by Cheng et al. (ibid.). In particular, the intrinsic growth hormone secretagogue activity of a compound which may be used in the present invention may be determined by this assay.

The term "patient" means an animal, such as a human, a companion animal such as a dog, cat and horse, and livestock such as cattle, swine and sheep. Particularly preferred patients are mammals, including both males and females, with humans being even more preferred.

The term "pharmaceutically acceptable" means that a substance or mixture of substances must be compatible with the other ingredients of a formulation, and not deleterious to the patient.

The terms "treating", "treat" or "treatment" include preventive (e.g., prophylactic) and palliative treatment.

The term "therapeutically effective amount" means an amount of a growth hormone secretagogue that ameliorates, attenuates, or eliminates a particular disease or condition associated with growth hormone secretion and/or production, or prevents or delays the onset of a disease or condition associated with growth hormone secretion and/or production.

The phrases "a compound of the present invention or a compound of Formula I" and the like, shall at all times be understood to include all active forms of such compounds, including, for example, the free form thereof, e.g., the free acid or base form, and also, all prodrugs, polymorphs, hydrates, solvates, stereoisomers, e.g., diastereomers and enantiomers, and the like, and all pharmaceutically acceptable salts as described above, unless specifically stated otherwise. It will also be appreciated that suitable active metabolites of such compounds are within the scope of the present invention.

The term "growth hormone deficient patient" (GHD patient) includes a patient, such as a young adult, who has growth hormone levels below 7 ng/ml. It also includes a patient, such as an elderly patient, who does not have "clinical" growth hormone deficiency as defined above, but who is recognized as being growth hormone deficient by other measures. For example, growth hormone deficient elderly patients have average growth hormone secretion which is often as much as 50 to 90% less than young adults; they have reduced average IGF-1 levels (an integrated measure of growth hormone secretion) of less than 50% of the values for young adults; they fail to have spontaneous growth hormone peaks above 5 ng/ml; and/or they have reduced sleep-associated rises in growth hormone. The decline of growth hormone secretion with aging is fully reviewed by E. Corpas, S.M. Harman, M.R. Blackman, "Human Growth Hormone and Human Aging," Endocrine Review, 14:20-39 (1993).

The term "a patient with age-related decline in physical performance" is a patient who, as he or she ages, exhibits objective evidence of decline in physical performance as measured by established methods of physical performance assessment. Measures of physical performance are objective tests of subjects' performance of standardized tasks, evaluated according to predetermined criteria that may include counting repetitions or timing the activity. A decline in physical performance results in increased risk of the patient suffering an adverse event such as an injurious fall and/or fracture. A decline in physical performance may also result in the patient having to be admitted to a nursing home and/or requiring assistance with carrying out activities of daily living.

Standardized tests of physical performance in older adults have been employed with increasing frequency in recent years. For example, J.M. Guralnik, et al., Journal of Gerontology, 49(2):M85-M94 (1994), and J.M. Guralnik et al., New England Journal of Medicine, 332:556-561 (1995), describe a short battery of physical performance tests used to assess lower extremity function in older adults. In M.E. Tinetti, et al., Journal of the American Medical Association, 273(17):1348-1353 (1995), physical performance skills in older adults were assessed through a series of qualitative and timed tests, described therein.

J.O. Judge et al., Journal of the American Geriatrics Society, 44: 1332-1341 (1996), describes measures of physical performance, such as hand grip strength, chair rise time, balance and gait speed, and found these physical performance measures were strongly associated with independence in Instrumental Activities of Daily Living (IADL). In D.B. Reuben et al., Journal of the American Geriatrics Society, 43: 17-23 (1995), a variety of measures of physical functioning were used, such as the Functional Status Questionnaire (FSQ), Katz Activities of Daily Living (ADL), the Older Americans Resources and Services Instrumental Activities of Daily Living (OARS-IADL), Physical Performance Test (PPT) and the Medical Outcomes Study SF-36, which were described therein. Also, workplace effectiveness tests may be used to assess one's ability to perform in the workplace. Other physical performance tests, which are known in the art, may be used to assess physical performance. In addition, any composite scoring system, which is composed of the results from a combination of any of the above tests, may be utilized to asses physical performance.

The term "functional health status" denotes a patient's functional capacity and/or capability given his or her current state of health or as affected by his or her current health state. A patient's functional health status may be determined by an assessment of certain measurable components, such as the following: strength, mobility, balance, physical activity levels, social activity levels, instrumental activities of daily living (IADL), general well-being, health-related quality of life, energy level, mood, sleep quality, cognitive status, mental acuity and ability to perform in the workplace. These components have been shown to be associated with a preservation of functional independence for a patient. Improvement in the above components may be measured by established methods known in the art.

In addition, to the physical performance measures cited above, there are other examples in the art of existing instruments that have been used to assess improvement in the above components. For instance, G. McGauley et al., Horm. Res., 45:34-37 (1996), and P.V. Carroll et al., European Journal of Endocrinology, 137: 146-153 (1997), used the Nottingham Health Profile (NHP) and/or the Psychological General Well-Being Schedule (PGWB) questionnaires to measure quality of life in adults that had been rendered GHD in adult life. The General Health Questionnaire (GHQ) was also used in such studies.

S.P. McKenna and L.C. Doward, PharmacoEconomics, 6(5):434-441 (1994), describes the development of a measure specifically designed to assess the quality of life of GH-deficient adults. Other more traditional instruments described therein include the Nottingham Health Profile (NHP), the Sickness Impact Profile (SIP), the General Health Questionaire (GHQ) and the RAND 36 Item Health Survey 1.0 (also known as the SF-36).

M.E. Wallymahmed et al., Clinical Endocrinology, 44: 403-411 (1996), describes the development of a health related quality of life model for use with adults with GH deficiency. Four previously validated scales were chosen for inclusion in that health related QOL model: the Nottingham Health Profile (NHP), the Hospital Anxiety and Depression Scale (HAD), The Self-Esteem Scale and The Mental Fatigue Questionnaire (MFQ). In addition, the Life Fulfilment Scale and the Impact Scale were adapted for use in this patient population.

This particular application of growth hormone secretagogues provides benefits relative to the administration of exogenous growth hormone. In particular, the growth hormone secretagogue enhances the normal pulsatile release of endogenous growth hormone and thus is more likely to reproduce the natural pattern of endogenous growth hormone release (see J. Clin. Endocrinol. Metab. 81: 4249-4257, 1996). Growth hormone secretagogues which are orally active also have the benefit of being able to be administered orally, rather than just intravenously, intraperitoneally or subcutaneously.

In view of their use according to the present invention, the growth hormone secretagogues of the present invention may be formulated into various pharmaceutical forms for administration purposes. A growth hormone secretagogue may be administered, alone or in combination, by oral, parenteral (e.g., intramuscular, intraperitoneal, intravenous or subcutaneous injection, or implant), nasal, vaginal, rectal, sublingual, or topical routes of administration and can be formulated with pharmaceutically acceptable carriers to provide dosage forms appropriate for each route of administration.

Solid dosage forms for oral administration include capsules, tablets, pills, powders and granules and for companion animals the solid dosage forms include an admixture with food and chewable forms. In such solid dosage forms, the compounds and combinations of this invention can be admixed with at least one inert pharmaceutically acceptable carrier such as sucrose, lactose, or starch. Such dosage forms can also comprise, as is normal practice, additional substances other than such inert diluents, e.g., lubricating agents such as magnesium stearate. In the case of capsules, tablets and pills, the dosage forms may also comprise buffering agents. Tablets and pills can additionally be prepared with enteric coatings. In the case of chewable forms, the dosage form may comprise flavoring agents and perfuming agents.

Liquid dosage forms for oral administration include pharmaceutically acceptable emulsions, solutions, suspensions, syrups and elixirs containing inert diluents commonly used in the art, such as water. Besides such inert diluents, compositions can also include adjuvants, such as wetting agents, emulsifying and suspending agents, and sweetening, flavoring and perfuming agents.

Preparations according to this invention for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized by, for example, filtration through a bacteria-retaining filter, by incorporating sterilizing agents into the compositions, by irradiating the compositions, or by heating the compositions. They can also be manufactured in the form of sterile solid compositions which can be dissolved in sterile water, or some other sterile injectable medium immediately before use.

Compositions for rectal or vaginal administration are preferably suppositories which may contain, in addition to a compound of the present invention, excipients such as cocoa butter or a suppository wax. Compositions for nasal or sublingual administration are also prepared with standard excipients well known in the art.

The dosage of a compound of the present invention in the compositions, methods and combinations of the present invention invention may be varied; however, it is necessary that the amount of the compound be such that a suitable dosage form is obtained. The selected dosage depends upon the desired therapeutic effect, on the route of administration, and on the duration of the treatment. Generally, dosage levels of between 0.0001 to 100 mg/kg of body weight daily are administered to humans and other animals, e.g., mammals, to obtain effective release of growth hormone. A preferred dosage range in humans is 0.01 to 5.0 mg/kg of body weight daily which can be administered as a single dose or divided into multiple doses.

A preferred dosage range in animals other than humans is 0.01 to 10.0 mg/kg of body weight daily which can be administered as a single dose or divided into multiple doses. A more preferred dosage range in animals other than humans is 0.1 to 5 mg/kg of body weight daily which can be administered as a single dose or divided into multiple doses.

Where the tartrate salt or other pharmaceutically acceptable salt of a compound of the present invention is used, the skilled person will be able to calculate effective dosage amounts by calculating the molecular weight of the salt form and performing simple stoichiometric ratios.

Also, the present invention includes within its scope the use of a growth hormone secretagogue according to the present invention, alone or in combination with another growth hormone secretagogue, such as those referenced herein, including the growth hormone releasing peptides GHRP-6 and GHRP-1 (described in U.S. Patent No. 4,411,890 and International Patent Applications, Publication Nos. WO 89/07110, WO 89/07111), GHRP-2 (described in WO 93/04081) and B-HT920, as well as hexarelin and growth hormone releasing hormone (GHRH, also designated GRF) and its analogs, growth hormone, recombinant or natural, and its analogs and somatomedins including IGF-I and IGF-II, or in combination with other therapeutic agents, such as α-adrenergic agonists such as clonidine or serotonin 5-HT1D agonists such as sumatriptan, or agents which inhibit somatostatin or its release such as physostigmine and pyridostigmine. Preferably, the growth hormone secretagogue may be used in combination with growth hormone releasing factor, an analog of growth hormone releasing factor, IGF-I or IGF-II. Also, the present invention includes within its scope the use of a growth hormone secretagogue according to the present invention, alone or in combination with another therapeutic agent, such as arginine, insulin or L-dopa optionally together with propranolol.

Methods to obtain the growth hormone releasing peptides GHRP-6 and GHRP-1 are described in U.S. Patent Nos. 4,411, 890 and PCT Patent Publications WO 89/07110, WO 89/07111, methods to obtain the growth hormone releasing peptide GHRP-2 are described in PCT Patent Publication WO 93/04081, and methods to obtain hexarelin are described in J. Endocrin. Invest., 15 (Suppl. 4), 45 (1992), all of which are incorporated herein by reference.

In addition, the present invention includes within its scope the use of a growth hormone secretagogue according to the present invention, alone or in combination with a growth promoting or anabolic agent, including TRH, PTH, diethylstilbesterol, estrogens, β-agonists, theophylline, anabolic steroids, enkephalins, E series prostaglandins, compounds disclosed in U.S. Patent No. 3,239,345, the disclosure of which is hereby incorporated by reference, e.g., zeranol; compounds disclosed in U.S. Patent No. 4,036,979, the disclosure of which is hereby incorporated by reference, e.g., sulbenox; and peptides disclosed in U.S. Patent No. 4,411,890, the disclosure of which is hereby incorporated by reference.

Also, the present invention includes within its scope the use of a growth hormone secretagogue according to the present invention, alone or in combination with an antidepressant, a prodrug thereof or a pharmaceutically acceptable salt of said antidepressant or said prodrug. Any antidepressant may be used in the methods of the present invention. The term antidepressant means an agent used to treat affective or mood disorders and related conditions. Affective mood disorders are characterized by changes in mood as the primary clinical manifestation. Either extreme of mood may be associated with psychosis, manifested as disordered or delusional thinking and perceptions which are often incongruent with the predominant mood. Affective disorders include major depression and mania, including bipolar manic-depressive illness. Preferred classes of antidepressants include norepinephrine reuptake inhibitors (NERIs), including secondary and tertiary amine tricyclics; selective serotonin reuptake inhibitors (SSRIs); combined NERI/SSRIs; monoamine oxidase (MAO) inhibitors; and atypical antidepressants.

Any norepinephrine reuptake inhibitor (NERI) may be used in the methods of the present invention. The term norepinephrine reuptake inhibitor means agents which potentiate the actions of biogenic amines by blocking their major means of physiological inactivation, which involves transport or reuptake into nerve terminals, and specifically, agents which block the reuptake of norepinephrine into said nerve terminals.

Preferred tertiary amine tricyclic norepinephrine reuptake inhibitors which may be used in accordance with the present invention include, but are not limited to, amitriptyline, which may be prepared as described in United States Patent No. 3,205,264; chlomipramine, which may be prepared as described in United States Patent No. 3,467,650; doxepin, which may be prepared as described in United States Patent No. 3,420,851; imipramine, which may be prepared as described in United States Patent No. 2,554,736; and trimipramine, which may be prepared as described in Jacob and Messer, *Compt. Rend.* 252, 2117 (1961).

Preferred secondary amine tricyclic norepinephrine reuptake inhibitors which may be used in accordance with the present invention include, but are not limited to, amoxapine, which may be prepared as described in United States Patent No. 3,663,696; desipramine, which may be prepared as described in United States Patent No. 3,454,554; maprotiline, which may be prepared as described in United States Patent No. 3,999,201; nortriptyline, which may be prepared as described in United States Patent No. 3,442,949; and protriptyline, which may be prepared as described in United States Patent No. 3,244,748.

Any selective serotonin reuptake inhibitor (SSRI) may be used in the methods of the present invention. The term selective serotonin reuptake inhibitor refers to a compound which inhibits the reuptake of serotonin by afferent neurons. Such inhibition is readily determined by those skilled in the art according to standard assays such as those disclosed in U.S. 4,536,518 and other U.S. patents recited in the next paragraph.

Preferred selective serotonin reuptake inhibitors (SSRIs) which may be used in accordance with the present invention include, but are not limited to: citalopram, which may be prepared as described in United States Patent No. 4,136,193; femoxetine, which may be prepared as described in United States Patent No. 3,912,743; fluoxetine, which may be prepared as described in United States Patent No. 4,314,081; fluvoxamine, which may be prepared as described in United States Patent No. 4,085,225; indalpine, which may be prepared as described in United States Patent No. 4,064,255; indeloxazine, which may be prepared as described in United States Patent No. 4,109,088; milnacipran, which may be prepared as described in United States Patent No. 4,478,836; paroxetine, which may be prepared as described in United States Patent No. 3,912,743 or United States Patent No. 4,007,196; sertraline and the hydrochloride salt of sertraline, which may be prepared as described in United States Patent No. 4,536,518; sibutramine, which may be prepared as described in United States Patent No. 4,929,629; and zimeldine, which may be prepared as described in United States Patent No. 3,928,369. Fluoxetine is also known as Prozac® . Sertraline hydrochloride is also known as Zoloft® . Sibutramine is also known as Meridia® .

Any combined NERI/SSRI may be used in the methods of the present invention. The term combined NERI/SSRI refers to a compound which blocks the reuptake of both serotonin and norepinephrine by afferent neurons. A preferred combined NERI/SSRI which may be used in accordance with the present invention is venlafaxine, which may be prepared as described in United States Patent No. 4,535,186.

Any monoamine oxidase (MAO) inhibitor may be used in the methods of the present invention. The term monoamine oxidase inhibitor refers to a compound which inhibits monoamine oxidase, for example, by blocking the metabolic deamination of a variety of monoamines by mitochondrial monoamine oxidase. Preferred monoamine oxidase inhibitors, which may be used in accordance with the present invention, include, but are not limited to, phenelzine, which may be prepared as described in United States Patent No. 3,000,903; tranylcypromine, which may be prepared as described in United States Patent No. 2,997,422; and selegiline, which may be prepared as described in United States Patent No. 4,564,706.

Any atypical antidepressant may be used in the methods of the present invention. The term atypical antidepressant refers to any antidepressant not within any of the aforesaid classes of antidepressants. Preferred atypical antidepressants which may be used in accordance with the present invention include, but are not limited to, bupropion, which may be prepared as described in United States Patent No. 3,885,046; nefazodone, which may be prepared as described in United States Patent No. 4,338,317; and trazodone, which may be prepared as described in United States Patent No. 3,381,009.

Also, the present invention includes within its scope the use of a growth hormone secretagogue according to the present invention, alone or in combination with an antipsychotic agent, a prodrug thereof or a pharmaceutically acceptable salt of said antipsychotic or said prodrug. Antipsychotic agents are used primarily in the management of patients with psychotic or other serious psychiatric illnesses marked by agitation and impaired reasoning. These drugs have other properties that are useful clinically, including antiemetic and antihistaminic effects and the ability to potentiate analgesics, sedatives, and general anesthetics. Effective antipsychotic compounds include phenothiazines, thioxanthenes, and heterocyclic dibenzazepines; butyrophenones (phenylbutylpiperidines) and diphenylbutylpiperidines; and indolones and other heterocyclic compounds. Chlorpromazine (Thorazine®) is the oldest representative of the phenothiazine-thioxanthene group of antipsychotic agents, and haloperidol (Haldol®) is the original butyrophenone and representative of several related classes of aromatic butylpiperidine derivatives. Further information on antipsychotic agents is provided in Goodman & Gilman's The Pharmacological Basis of Therapeutics, Ninth Edition, McGraw-Hill, New York (1996), pages 399-430, which is incorporated by reference herein. Examples of other antipsychotic agents include clozapine (Clozaril®), loxapine (Loxitane®), molindone hydrochloride (Moban®), thiothixene (Navane®), olanzapine (Zyprexa®), ziprasidone and its hydrochloride salt (Zeldox® or Geodon®), prochlorperazine (Compazine®), perphenazine (Trilafon®), trifluoperazine hydrochloride (Stalazine®) and risperidone (Risperdal®) (Physician's Desk Reference, 53^{rd} Ed., Medical Economics Co., Inc., Montvale, N.J. (1999)).

Also, the present invention includes within its scope the use of a growth hormone secretagogue according to the present invention, alone or in combination with an antianxiety agent, a prodrug thereof or a pharmaceutically acceptable salt of said antianxiety agent or said prodrug. Currently, benzodiazepines are the most commonly employed antianxiety agents for generalized anxiety disorder. Some benzodiazepines (alprazolam, clonazepam and lorazepam) are effective in severe anxiety with strong autonomic overactivity (panic disorder) as are several antidepressant agents. For generalized or nonspecific anxiety, the specific agent selected seems to make little difference. In the elderly orin patients with impaired hepatic function, oxazepam in small, divided doses is currently favored due to its brief action and direct conjugation and elimination. Benzodiazepines commonly are given to outpatients with anxiety mixed with symptoms of depression, although their specific efficacy in the core features of severe major depression is not well demonstrated. Potent benzodiazepines also are commonly employed, adjunctively, in the short-term management of acutely psychotic or manic patients. Further information on antianxiety agents is provided in Goodman & Gilman's The Pharmacological Basis of Therapeutics, Ninth Edition, McGiaw-Hill, New York (1996), pages 420-430, which is incorporated by reference herein. Examples of antianxiety agents include lorazepam (Ativan®), chlordiazepoxide hydrochloride (Librium®), oxazepam (Serax®), diazepam (Valium®), alprazolam (Xanax®), buspirone hydrochloride (BuSpar®), doxepin hydrochloride (Sinequan®), hydroxyzine pamoate (Vistaril®), and clonazepam (Klonopin®) (Physician's Desk Reference, 53^{rd} Ed., Medical Economics Co., Inc., Montvale, N.J. (1999)).

Also, the present invention includes within its scope the use of a growth hormone secretagogue according to the present invention, alone or in combination with a naturaceutic, a prodrug thereof or a pharmaceutically acceptable salt of said naturaceutic or said prodrug. A naturaceutic is typically an over-the-counter composition that is promoted as, for example, improving health or general well-being, enhancing mood, improving mental acuity or memory. It includes herbal remedies, such as ginko biloba, St. John's Wart, valerian (from heliotrope) and melatonin.

The disclosures of each of the references, patents and published applications cited within this description are hereby incorporated by reference herein.

The present invention includes within its scope the use of a pharmaceutical composition according to the present invention comprising, as an active ingredient, at least one growth hormone secretagogue in association with a pharmaceutical carrier, vehicle or diluent. The present invention also includes within its scope the use of a compound of Formula I for the preparation of a medicament for the uses disclosed herein.

Combinations of these therapeutic agents, some of which have been mentioned herein, with a growth hormone secretagogue may bring additional complementary properties to enhance the desirable properties of these various therapeutic agents. The growth hormone secretagogue and the other agent may be co-administered, either in concomitant therapy or in a fixed combination. In these combinations, the growth hormone secretagogue and the other therapeutic agent(s) may be independently present in the dose ranges from 0.01 to 1 times the dose levels which are effective when these compounds and secretagogues are used singly.

Typically, the individual daily dosages for these combinations may range from about one-fifth of the minimally recommended clinical dosages to the maximum recommended levels for the entities when they are given singly. These dose ranges may be adjusted on a unit basis as necessary to permit divided daily dosage and, as noted above, the dose will vary depending on the nature and severity of the disease, the weight of the patient, special diets and other factors.

These combinations may be formulated into pharmaceutical compositions as known in the art and as discussed herein. Since the present invention has an aspect that relates to treatment with a combination of active ingredients which may be administered separately, the invention also relates to combining separate pharmaceutical compositions in kit form. The kit comprises two separate pharmaceutical compositions: a growth hormone secretagogue, a prodrug thereof or a pharmaceutically acceptable salt of said growth hormone secretagogue or said prodrug; and a second therapeutic agent as described herein. The kit comprises a container for containing the separate compositions such as a divided bottle or a divided foil packet, however, the separate compositions may also be contained within a single, undivided container. Typically, the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably, the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, e.g., in the form of numbers next to the tablets or capsules whereby the numbers correspond with the days of the regimen which the dosage form so specified should be ingested. Another example of such a memory aid is a calendar printed on the card e.g., as follows "First Week, Monday, Tuesday, ...etc.... Second Week, Monday, Tuesday,..." etc. Other variations of memory aids will be readily apparent. A "daily dose" can be a single tablet or capsule or several tablets or capsules to be taken on a given day. Also, a daily dose of a second therapeutic agent as described herein can consist of one tablet or capsule while a daily dose of the growth hormone secretagogue, prodrug thereof or pharmaceutically acceptable salt of said growth hormone secretagogue or said prodrug can consist of several tablets or capsules or vice versa. The memory aid should reflect this.

In another specific embodiment of the invention, a dispenser designed to dispense the daily doses one at a time in the order of their intended use is provided. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

The utility of the compounds described herein in the methods of the present invention are demonstrated by their activity in one or more of the assays described below.

### Assay for Stimulation of Growth Hormone Release from Rat Pituicytes

Compounds having the ability to stimulate GH secretion from cultured rat pituitary cells are identified using the following protocol. This test is also useful for comparison to standards to determine dosage levels.

Cells are isolated from pituitaries of 6-week old male Wistar rats. Following decapitation, the anterior pituitary lobes are removed into cold, sterile Hank's balanced salt solution without calcium or magnesium (HBSS). Tissues are finely minced, then subjected to two cycles of mechanically assisted enzymatic dispersion using 10 U/mL bacterial protease (EC 3.4.24.4, Sigma P-6141, St. Louis, Missouri) in HBSS. The tissue-enzyme mixture is stirred in a spinner flask at 30 rpm in a 5% CO₂ atmosphere at 37 °C for 30 min., with manual trituration after 15 min. and 30 min. using a 10-mL pipet. This mixture is centrifuged at 200 x g for 5 min. Horse serum (35% final concentration) is added to the supernatant to neutralize excess protease. The pellet is resuspended in fresh protease (10 U/mL), stirred for about 30 min. more under the previous conditions, and manually triturated, ultimately through a 23-gauge needle. Again, horse serum (35% final concentration) is added, then the cells from both digests are combined, pelleted (200 x g for about 15 min.), resuspended in culture medium (Dulbecco's Modified Eagle Medium (D-MEM) supplemented with 4.5 g/L glucose, 10% horse serum, 2.5% fetal bovine serum, 1% non-essential amino acids, 100 U/mL nystatin and 50 mg/mL gentamycin sulfate, Gibco, Grand Island, New York) and counted. Cells are plated at 6.0-6.5x10⁴ cells per cm² in 48-well Costar™ (Cambridge, Massachusetts) dishes and cultured for 3-4 days in culture medium.

Just prior to carrying out a GH secretion assay, culture wells are rinsed twice with release medium, then equilibrated for 30 minutes in release medium (D-MEM buffered with 25 mM Hepes, pH 7.4 and containing 0.5% bovine serum albumin at 37 °C). Test compounds are dissolved in DMSO, then diluted into pre-warmed release medium. Assays are typically run in quadruplicate. The assay is initiated by adding 0.5 mL of release medium (with vehicle or test compound) to each culture well. Incubation is carried out at 37 °C for 15 minutes, then terminated by removal of the release medium, which is centrifuged at 2000 x g for 15 minutes to remove cellular material. Rat growth hormone concentrations in the supernatants are determined by a standard radioimmunoassay protocol described below.

### Assay for Exogenously-Stimulated Growth Hormone Release in the Rat after Intravenous Administration of Test Compounds

Twenty-one day old female Sprague-Dawley rats (Charles River Laboratory, Wilmington, MA) are allowed to acclimate to local vivarium conditions (24 °C, 12 hr light, 12 hr dark cycle) for approximately 1 week before testing of a compound of this invention. All rats are allowed access to water and a pelleted commercial diet (Agway Country Food, Syracuse NY) *ad libitum.*

On the day of the experiment, test compounds are dissolved in vehicle containing 1% ethanol, 1 mM acetic acid and 0.1% bovine serum albumin in saline. Each test is conducted in three rats. Rats are weighed and anesthetized via intraperitoneal injection of sodium pentobarbital (Nembutol®, 50 mg/kg body weight). Fourteen minutes after anesthetic administration, a bicod sample is taken by nicking the tip of the tail and allowing the blood to drip into amicrocentrifuge tube (baseline blood sample, approximately 100 µl). Fifteen minutes after anesthetic administration, a test compound is delivered by intravenous injection into the tail vein, with a total injection volume of 1 mL/kg body weight. Additional blood samples are taken from the tail at 5, 10 and 15 minutes after administration of a compound of this invention. Blood samples are kept on ice until serum separation by centrifugation (1430 x g for 10 minutes at 10°C). Serum is stored at -80 °C until serum growth hormone determination by radioimmunoassay as described below.

### Measurement of Rat Growth Hormone

Rat growth hormone concentrations are determined by double antibody radioimmunoassay using a rat growth hormone reference preparation (NIDDK-rGH-RP-2) and rat growth hormone antiserum raised in monkey (NIDDK-anti-rGH-S-5) obtained from Dr. A. Parlow (Harbor-UCLA Medical Center, Torrance, CA). Additional rat growth hormone (1.5U/mg, #G2414, Scripps Labs, San Diego, CA) is iodinated to a specific activity of approximately 30 µCi/µg by the chloramine T method for use as tracer. Immune complexes are obtained by adding goat antiserum to monkey IgG (ICN/Cappel, Aurora, OH) plus polyethylene glycol, MW 10,000-20,000 to a final concentration of 4.3%; recovery is accomplished by centrifugation according to methods well known to those skilled in the art. This assay has a working range of 0.08-2.5 µg rat growth hormone per tube.

### Assessment of Growth Hormone Release in the Dog after Oral Administration

On the day of dosing, the test compound is weighed out for the appropriate dose and dissolved in water. Doses are delivered at a volume of 0.5-3 mL/kg by oral gavage to 2-4 dogs for each dosing regimen. Blood samples (5 mL) are collected from the jugular vein by direct venipuncture pre-dose and at 0.17, 0.33, 0.5, 0.75, 1, 2, 4, 6, 8 and 24 hours post dose using 5 mL vacutainers containing lithium heparin. The prepared plasma is stored at -20 °C until analysis.

### Measurement of Canine Growth Hormone

Canine growth hormone concentrations are determined by a standard radioimmunoassay protocol using canine growth hormone (antigen for iodination and reference preparation AFP-1983B) and canine growth hormone antiserum raised in monkey (AFP-21452578) obtained from Dr. A. Parlow (Harbor-UCLA Medical Center, Torrence, CA). Tracer is produced by chloramine T-iodination of canine growth hormone to a specific activity of 20-40 µCi/µg. Immune complexes are obtained by adding goat antiserum to monkey IgG (ICN/Cappel, Aurora, OH) plus polyethylene glycol, MW 10,000-20,000 to a final concentration of 4.3%; recovery is accomplished by centrifugation according to methods well known to those skilled in the art. This assay has a working range of 0.08-2.5 µg canine GH/tube.

### Assessment of Canine Growth Hormone and Insulin-Like Growth Factor-1 Levels in the Dog after Chronic Oral Administration

The dogs receive test compound daily for either 7 or 14 days. Each day of dosing, the test compound is weighed out for the appropriate dose and dissolved in water. Doses are delivered at a volume of 0.5-3 ml/kg by gavage to 5 dogs for each dosing regimen. Blood samples are collected at days 0, 3, 7, 10 and 14. Blood samples (5 ml) are obtained by direct venipuncture of the jugular vein at pre-dose, 0.17, 0.33, 0.5, 0.75, 1, 2, 3, 6, 8, 12 and 24 hours post administration on days 0, 7 and 14 using 5 ml vacutainers containing lithium heparin for GH determination. In addition, blood is drawn pre-dose and 8 hours after dosing on days 3 and 10 for IGF-I determination. The prepared plasma is stored at -20 °C until analysis.

Plasma samples are extracted with acid ethanol (0.25N HCI in 90% ethanol), centrifuged, then the supernatant is neutralized with tris[hydroxymethyl]amino-methane (registered name is TRIZMA base, manufactured by Sigma Chemical Co.) prior to determination of IGF-I concentration using the Nichols Institute IGF-I extraction kit (San Juan Capistrano, CA).

While the foregoing description discloses the present invention, with examples provided for the purpose of illustration, it will be understood that the practice of the present invention encompasses all of the usual variations, adaptations or modifications as come within the scope of the following claims and their equivalents.

## Claims

1. Use of a therapeutically effective amount of a growth hormone secretagogue for the preparation of a medicament for improving functional health status in a patient in need thereof.

2. A use of claim 1 wherein the instrumental activities of daily living of the patient are improved.

3. A use of claim 1 wherein the energy level, mood or sleep quality of the patient is improved.

4. A use of claim 1 wherein the cognitive status of the patient is improved.

5. A use of claim 1 wherein the health-related quality of life of the patient is improved.

6. A use of claim 1 wherein the patient is a human who is elderly, chronically ill, has age-related decline in physical performance or is growth hormone deficient.

7. A use of claim 1 wherein said growth hormone secretagogue is a compound of the Formula I: 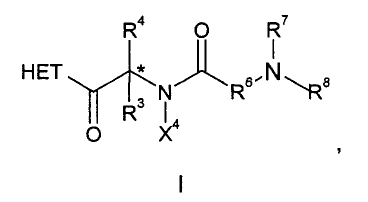 or a stereoisomeric mixture thereof, diastereomerically enriched, diastereomerically pure, enantiomerically enriched or enantiomerically pure isomer thereof, or a prodrug of such compound, mixture or isomer thereof, or a pharmaceutically acceptable salt of the compound, mixture, isomer or prodrug, or a tautomer thereof,
wherein:
HET is a heterocyclic moiety selected from the group consisting of 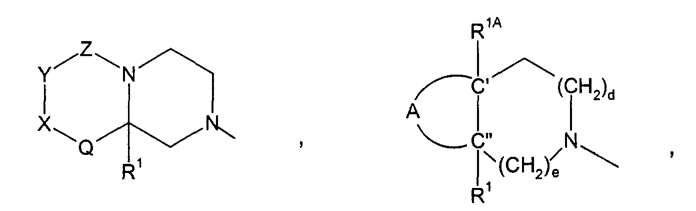 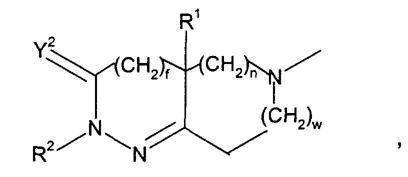 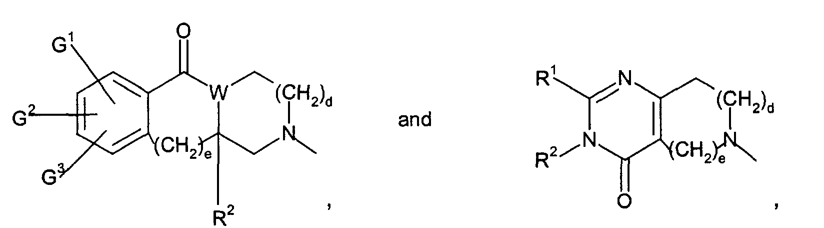
d is 0, 1 or 2;
e is 1 or 2;
f is 0 or 1;
n and w are 0, 1 or 2, provided that n and w cannot both be 0 at the same time;
Y² is oxygen or sulfur;
A is a divalent radical, where the left hand side of the radical as shown below is connected to C" and the right hand side of the radical as shown below is connected to C', selected from the group consisting of
-NR²-C(O)-NR²-, -NR²-S(O)₂-NR²-, -O-C(O)-NR²-, -NR²-C(O)-O-, -C(O)-NR²-C(O)-, -C(O)-NR²-C(R⁹R¹⁰)-, -C(R⁹R¹⁰)-NR²-C(O)-, -C(R⁹R¹⁰)-C(R⁹R¹⁰)-C(R⁹R¹⁰)-, -S(O)₂-C(R⁹R¹⁰)-C(R⁹R¹⁰)-, -C(R⁹R¹⁰)-O-C(O)-, -C(R⁹R¹⁰)-O-C(R⁹R¹⁰)-, -NR²-C(O)-C(R⁹R¹⁰)-, -O-C(O)-C(R⁹R¹⁰)-, -C(R⁹R¹⁰)-C(O)-NR²-, -C(R⁹R¹⁰)-C(O)-O-, -C(O)-NR²-C(R⁹R¹⁰)-C(R⁹R¹⁰)-, -C(O)-O-C(R⁹R¹⁰)-, -C(R⁹R¹⁰)-C(R⁹R¹⁰)-C(R⁹R¹⁰)-C(R⁹R¹⁰)-, -S(O)₂-NR²-C(R⁹R¹⁰)-C(R⁹R¹⁰)-, -C(R⁹R¹⁰)-C(R⁹R¹⁰)-NR²-C(O)-, -C(R⁹R¹⁰)-C(R⁹R¹⁰)-O-C(O)-, -NR²-C(O)-C(R⁹R¹⁰)-C(R⁹R¹⁰)-, -NR²-S(O)₂-C(R⁹R¹⁰)-C(R⁹R¹⁰)-, -O-C(O)-C(R⁹R¹⁰)-C(R⁹R¹⁰)-, -C(R⁹R¹⁰)-C(R⁹R¹⁰)-C(O)-NR²-, -C(R⁹R¹⁰)-C(R⁹R¹⁰)-C(O)-, -C(R⁹R¹⁰)-NR²-C(O)-O-, -C(R⁹R¹⁰)-O-C(O)-NR², -C(R⁹R¹⁰)-NR²-C(O)-NR²-, -NR²-C(O)-O-C(R⁹R¹⁰)-, -NR²-C(O)-NR²-C(R⁹R¹⁰)-, -NR²-S(O)₂-NR²-C(R⁹R¹⁰)-, -O-C(O)-NR²-C(R⁹R¹⁰)-, -C(O)-N=C(R¹¹)-NR²-, -C(O)-NR²-C(R¹¹)=N-, -C(R⁹R¹⁰)-NR¹²-C(R⁹R¹⁰)-, -NR¹²-C(R⁹R¹⁰)-, -NR¹²-C(R⁹R¹⁰)-C(R⁹R¹⁰)-, -C(O)-O-C(R⁹R¹⁰)-C(R⁹R¹⁰)-, -NR²-C(R¹¹)=N-C(O)-, -C(R⁹R¹⁰)-C(R⁹R¹⁰)-N(R¹²)-, -C(R⁹R¹⁰)-NR¹²-, -N=C(R¹¹)-NR²-C(O)-, -C(R⁹R¹⁰)-C(R⁹R¹⁰)-NR²-S(O)₂-, -C(R⁹R¹⁰)-C(R⁹R¹⁰)-S(O)₂-NR²-, -C(R⁹R¹⁰)-C(R⁹R¹⁰)-C(O)-O-, -C(R⁹R¹⁰)-S(O)₂-C(R⁹R¹⁰)-, -C(R⁹R¹⁰)-C(R⁹R¹⁰)-S(O)₂-,-O-C(R⁹R¹⁰)-C(R⁹R¹⁰)-, -C(R⁹R¹⁰)-C(R⁹R¹⁰)-O-, -C(R⁹R¹⁰)-C(O)-C(R⁹R¹⁰)-, -C(O)-C(R⁹R¹⁰)-C(R⁹R¹⁰)- and -C(R⁹R¹⁰)-NR²-S(O)₂-NR²-;
Q is a covalent bond or CH₂;
W is CH or N;
X is CR⁹R¹⁰, C=CH₂ or C=O;
Y is CR⁹R¹⁰, O or NR²;
Z is C=O, C=S or S(O)₂;
G¹ is hydrogen, halo, hydroxy, nitro, amino, cyano, phenyl, carboxyl, -CONH₂, -(C₁-C₄)alkyl optionally independently substituted with one or more phenyl, one or more halogens or one or more hydroxy groups, -(C₁-C₄)alkoxy optionally independently substituted with one or more phenyl, one or more halogens or one or more hydroxy groups, -(C₁-C₄)alkylthio, phenoxy, -COO(C₁-C₄)alkyl, N,N-di-(C₁-C₄)alkylamino,-(C₂-C₆)alkenyl optionally independently substituted with one or more phenyl, one or more halogens or one or more hydroxy groups, -(C₂-C₆)alkynyl optionally independently substituted with one or more phenyl, one or more halogens or one or more hydroxy groups, -(C₃-C₆)cycloalkyl optionally independently substituted with one or more (C₁-C₄)alkyl groups, one or more halogens or one or more hydroxy groups, -(C₁-C₄)alkylamino carbonyl or di-(C₁-C₄)alkylamino carbonyl;
G² and G³ are each independently selected from the group consisting of hydrogen, halo, hydroxy, -(C₁-C₄)alkyl optionally independently substituted with one to three halo groups and -(C₁-C₄)alkoxy optionally independently substituted with one to three halo groups;
R¹ is hydrogen, -CN, -(CH₂)_{q}N(X⁶)C(O)X⁶, -(CH₂)_{q}N(X⁶)C(O)(CH₂)ₜ-A¹, -(CH₂)_{q}N(X⁶)S(O)₂(CH₂)ₜ-A¹, -(CH₂)_{q}N(X⁶)S(O)₂X⁶, -(CH₂)_{q}N(X⁶)C(O)N(X⁶)(CH₂)ₜ-A¹, -(CH₂)_{q}N(X⁶)C(O)N(X⁶)(X⁶), -(CH₂)_{q}C(O)N(X⁶)(X⁶), -(CH₂)_{q}C(O)N(X⁶)(CH₂)ₜ-A¹, -(CH₂)_{q}C(O)OX⁶, -(CH₂)_{q}C(O)O(CH₂)ₜ-A¹, -(CH₂)_{q}OX⁶, -(CH₂)_{q}OC(O)X⁶, -(CH₂)_{q}OC(O)(CH₂)ₜ-A¹, -(CH₂)_{q}OC(O)N(X⁶)(CH₂)ₜ-A¹, -(CH₂)_{q}OC(O)N(X⁶)(X⁶), -(CH₂)_{q}C(O)X⁶, -(CH₂)_{q}C(O)(CH₂)ₜ-A¹, -(CH₂)_{q}N(X⁶)C(O)OX⁶, -(CH₂)_{q}N(X⁶)S(O)₂N(X⁶)(X⁶), -(CH₂)_{q}S(O)ₘX⁶, -(CH₂)_{q}S(O)ₘ(CH₂)ₜ-A¹, -(C₁-C₁₀)alkyl, -(CH₂)ₜ-A¹, -(CH₂)_{q}-(C₃-C₇)cycloalkyl, -(CH₂)_{q}-Y¹-(C₁-C₆)alkyl, -(CH₂)_{q}-Y¹-(CH₂)ₜ-A¹ or -(CH₂)_{q}-Y¹-(CH₂)ₜ-(C₃-C₇)cycloalkyl;
where the alkyl and cycloalkyl groups in the definition of R¹ are optionally substituted with (C₁-C₄)alkyl, hydroxy, (C₁-C₄)alkoxy, carboxyl, -CONH₂, -S(O)ₘ(C₁-C₆)alkyl, -CO₂(C₁-C₄)alkyl ester, 1H-tetrazol-5-yl or 1, 2 or 3 fluoro groups;
Y¹ is O, S(O)ₘ, -C(O)NX⁶-, -CH=CH-, -C≡C-, -N(X⁶)C(O)-, -C(O)NX⁶-, -C(O)O-, -OC(O)N(X⁶)- or -OC(O)-;
q is 0, 1, 2, 3 or 4;
t is 0, 1, 2 or 3;
said (CH₂)_{q} group and (CH₂)ₜ group in the definition of R¹ are optionally independently substituted with hydroxy, (C₁-C₄)alkoxy, carboxyl, -CONH₂, -S(O)ₘ(C₁-C₆)alkyl, -CO₂(C₁-C₄)alkyl ester, 1H-tetrazol-5-yl, 1, 2 or 3 fluoro groups or 1 or 2 (C₁-C₄)alkyl groups;
R^{1A} is selected from the group consisting of hydrogen, F, Cl, Br, I, (C₁-C₆)alkyl, phenyl(C₁-C₃)alkyl, pyridyl(C₁-C₃)alkyl, thiazolyl(C₁-C₃)alkyl and thienyl(C₁-C₃)alkyl, provided that R^{1A} is not F, Cl, Br or I when a heteroatom is vicinal to C";
R² is hydrogen, (C₁-C₈)alkyl, -(C₀-C₃)alkyl-(C₃-C₈)cycloalkyl, -(C₁-C₄)alkyl-A¹ or A¹;
where the alkyl groups and the cycloalkyl groups in the definition of R² are optionally substituted with hydroxy, -C(O)OX⁶, -C(O)N(X⁶)(X⁶), -N(X⁶)(X⁶),-S(O)ₘ(C₁-C₆)alkyl, -C(O)A¹, -C(O)(X⁶), CF₃, CN or 1, 2 or 3 independently selected halo groups;
R³ is selected from the group consisting of A¹, (C₁-C₁₀)alkyl, -(C₁-C₆)alkyl-A¹, -(C₁-C₆)alkyl-(C₃-C₇)cycloalkyl, -(C₁-C₅)alkyl-X¹-(C₁-C₅)alkyl, -(C₁-C₅)alkyl-X¹-(C₀-C₅)alkyl-A¹ and -(C₁-C₅)alkyl-X¹-(C₁-C₅)alkyl-(C₃-C₇)cycloalkyl;
where the alkyl groups in the definition of R³ are optionally substituted with -S(O)ₘ(C₁-C₆)alkyl, -C(O)OX³, 1, 2, 3, 4 or 5 independently selected halo groups or 1, 2 or 3 independently selected -OX³ groups; X¹ is O, S(O)ₘ, -N(X²)C(O)-, -C(O)N(X²)-, -OC(O)-, -C(O)O-, -CX²=CX²-, -N(X²)C(O)O-, -OC(O)N(X²)- or -C≡C-;
R⁴ is hydrogen, (C₁-C₆)alkyl or (C₃-C₇)cycloalkyl, or R⁴ is taken together with R³ and the carbon atom to which they are attached and form (C₅-C₇)cycloalkyl, (C₅-C₇)cycloalkenyl, a partially saturated or fully saturated 4- to 8-membered ring having 1 to 4 heteroatoms independently selected from the group consisting of oxygen, sulfur and nitrogen, or is a bicyclic ring system consisting of a partially saturated or fully saturated 5- or 6-membered ring, fused to a partially saturated, fully unsaturated or fully saturated 5- or 6-membered ring, optionally having 1 to 4 heteroatoms independently selected from the group consisting of nitrogen, sulfur and oxygen;
X⁴ is hydrogen or (C₁-C₆)alkyl or X⁴ is taken together with R⁴ and the nitrogen atom to which X⁴ is attached and the carbon atom to which R⁴ is attached and form a five to seven membered ring;
R⁶ is a bond or is 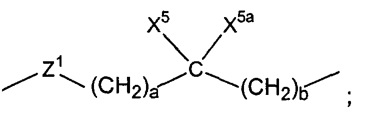
where a and b are each independently 0, 1, 2 or 3;
X⁵ and X^{5a} are each independently selected from the group consisting of hydrogen, CF₃, A¹ and optionally substituted (C₁-C₆)alkyl;
the optionally substituted (C₁-C₆)alkyl in the definition of X⁵ and X^{5a} is optionally substituted with a substituent selected from the group consisting of A¹, OX², -S(O)ₘ(C₁-C₆)alkyl, -C(O)OX², (C₃-C₇)cycloalkyl, -N(X²)(X²) and -C(O)N(X²)(X²);
or the carbon bearing X⁵ or X^{5a} forms one or two alkylene bridges with the nitrogen atom bearing R⁷ and R⁸ wherein each alkylene bridge contains 1 to 5 carbon atoms, provided that when one alkylene bridge is formed then only one of X⁵ or X^{5a} is on the carbon atom and only one of R⁷ or R⁸ is on the nitrogen atom and further provided that when two alkylene bridges are formed then X⁵ and X^{5a} cannot be on the carbon atom and R⁷ and R⁸ cannot be on the nitrogen atom;
or X⁵ is taken together with X^{5a} and the carbon atom to which they are attached and form a partially saturated or fully saturated 3- to 7-membered ring, or a partially saturated or fully saturated 4- to 8-membered ring having 1 to 4 heteroatoms independently selected from the group consisting of oxygen, sulfur and nitrogen;
or X⁵ is taken together with X^{5a} and the carbon atom to which they are attached and form a bicyclic ring system consisting of a partially saturated or fully saturated 5- or 6-membered ring, optionally having 1 or 2 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
Z¹ is a bond, O or N-X², provided that when a and b are both 0 then Z¹ is not N-X² or O;
or R⁶ is -(CR^{a}R^{b})ₐ-E-(CR^{a}R^{b})_{b}-, where the -(CR^{a}R^{b})ₐ- group is attached to the carbonyl carbon of the amide group of the compound of formula I and the -(CR^{a}R^{b})_{b} group is attached to the terminal nitrogen atom of the compound of formula I;
E is -O-, -S-, -CH=CH- or an aromatic moiety selected from 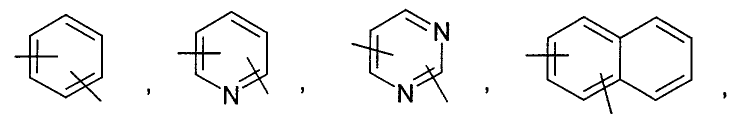 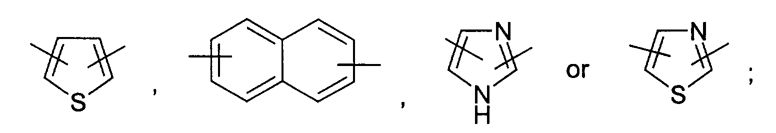 said aromatic moiety in the definition of E optionally substituted with up to three halo, hydroxy, -N(R^{c})(R^{c}), (C₁-C₆)alkyl or (C₁-C₆)alkoxy;
R^{a} and R^{b} are, for each occurrence, independently hydrogen, (C₁-C₆)alkyl, trifluoromethyl, phenyl or monosubstituted (C₁-C₆)alkyl where the substituents are imidazolyl, naphthyl, phenyl, indolyl, p-hydroxyphenyl, -OR^{c}, S(O)ₘR^{c}, C(O)OR^{c}, (C₃-C₇)cycloalkyl, -N(R^{c})(R^{c}), -C(O)N(R^{c})(R^{c}), or R^{a} or R^{b} may independently be joined to one or both of R⁷ or E (where E is other than O, S or -CH=CH-) to form an alkylene bridge between the terminal nitrogen and the alkyl portion of the R^{a} or R^{b} and the R⁷ or E group,
wherein the bridge contains 1 to 8 carbon atoms; or R^{a} and R^{b} may be joined to one another to form a (C₃-C₇)cycloalkyl;
R^{c}, for each occurrence, is independently hydrogen or (C₁-C₆)alkyl;
a and b are independently 0, 1, 2 or 3, with the proviso that if E is -O- or -S-, b is other than 0 or 1 and with the further proviso that if E is -CH=CH-, b is other than 0;
R⁷ and R⁸ are each independently hydrogen or optionally substituted (C₁-C₆)alkyl;
where the optionally substituted (C₁-C₆)alkyl in the definition of R⁷ and R⁸ is optionally independently substituted with A¹, -C(O)O-(C₁-C₆)alkyl, -S(O)ₘ(C₁-C₆)alkyl, 1 to 5 halo groups, 1 to 3 hydroxy groups, 1 to 3 -O-C(O)(C₁-C₁₀)alkyl groups or 1 to 3 (C₁-C₆)alkoxy groups; or
R⁷ and R⁸ can be taken together to form -(CH₂)ᵣ-L-(CH₂)ᵣ-;
where L is C(X²)(X²), S(O)ₘ or N(X²);
R⁹ and R¹⁰ are each independently selected from the group consisting of hydrogen, fluoro, hydroxy and (C₁-C₅)alkyl optionally independently substituted with 1-5 halo groups;
R¹¹ is selected from the group consisting of (C₁-C₅)alkyl and phenyl optionally substituted with 1-3 substitutents each independently selected from the group consisting of (C₁-C₅)alkyl, halo and (C₁-C₅)alkoxy;
R¹² is selected from the group consisting of (C₁-C₅)alkylsulfonyl, (C₁-C₅)alkanoyl and (C₁-C₅)alkyl where the alkyl portion is optionally independently substituted by 1-5 halo groups;
A¹ for each occurrence is independently selected from the group consisting of (C₅-C₇)cycloalkenyl, phenyl, a partially saturated, fully saturated or fully unsaturated 4- to 8-membered ring optionally having 1 to 4 heteroatoms independently selected from the group consisting of oxygen, sulfur and nitrogen and a bicyclic ring system consisting of a partially saturated, fully unsaturated or fully saturated 5- or 6-membered ring, optionally having 1 to 4 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
A¹ for each occurrence is independently optionally substituted, on one or optionally both rings if A¹ is a bicyclic ring system, with up to three substituents, each substituent independently selected from the group consisting of F, Cl, Br, I, OCF₃, OCF₂H, CF₃, CH₃, OCH₃, -OX⁶, -C(O)N(X⁶)(X⁶), -C(O)OX⁶, oxo, (C₁-C₆)alkyl, nitro, cyano, benzyl, -S(O)ₘ(C₁-C₆)alkyl, 1H-tetrazol-5-yl, phenyl, phenoxy, phenylalkyloxy, halophenyl, methylenedioxy, -N(X⁶)(X⁶), -N(X⁶)C(O)(X⁶), -S(O)₂N(X⁶)(X⁶), -N(X⁶)S(O)₂-phenyl, -N(X⁶)S(O)₂X⁶, -CONX¹¹X¹², -S(O)₂NX¹¹X¹², -NX⁶S(O)₂X¹², -NX⁶CONX¹¹X¹², -NX⁶S(O)₂NX¹¹X¹², -NX⁶C(O)X¹², imidazolyl, thiazolyl and tetrazolyl, provided that if A¹ is optionally substituted with methylenedioxy then it can only be substituted with one methylenedioxy;
where X¹¹ is hydrogen or optionally substituted (C₁-C₆)alkyl;
the optionally substituted (C₁-C₆)alkyl defined for X¹¹ is optionally independently substituted with phenyl, phenoxy, (C₁-C₆)alkoxycarbonyl, -S(O)ₘ(C₁-C₆)alkyl, 1 to 5 halo groups, 1 to 3 hydroxy groups, 1 to 3 (C₁-C₁₀)alkanoyloxy groups or 1 to 3 (C₁-C₆)alkoxy groups;
X¹² is hydrogen, (C₁-C₆)alkyl, phenyl, thiazolyl, imidazolyl, furyl or thienyl, provided that when X¹² is not hydrogen, the X¹² group is optionally substituted with one to three substituents independently selected from the group consisting of Cl, F, CH₃, OCH₃, OCF₃ and CF₃;
or X¹¹ and X¹² are taken together to form -(CH₂)ᵣ-L¹-(CH₂)ᵣ-;
L¹ is C(X²)(X²), O, S(O)ₘ or N(X²);
r for each occurrence is independently 1, 2 or 3;
X² for each occurrence is independently hydrogen, optionally substituted (C₁-C₆)alkyl or optionally substituted (C₃-C₇)cycloalkyl, where the optionally substituted (C₁-C₆)alkyl and optionally substituted (C₃-C₇)cycloalkyl in the definition of X² are optionally independently substituted with -S(O)ₘ(C₁-C₆)alkyl, -C(O)OX³, 1 to 5 halo groups or 1-3 OX³ groups;
X³ for each occurrence is independently hydrogen or (C₁-C₆)alkyl;
X⁶ for each occurrence is independently hydrogen, optionally substituted (C₁-C₆)alkyl, (C₂-C₆)halogenated alkyl, optionally substituted (C₃-C₇)cycloalkyl, (C₃-C₇)-halogenated cycloalkyl, where optionally substituted (C₁-C₆)alkyl and optionally substituted (C₃-C₇)cycloalkyl in the definition of X⁶ is optionally independently mono- or di-substituted with (C₁-C₄)alkyl, hydroxy, (C₁-C₄)alkoxy, carboxyl, CONH₂, -S(O)ₘ(C₁-C₆)alkyl, carboxylate (C₁-C₄)alkyl ester or 1 H-tetrazol-5-yl; or
when there are two X⁶ groups on one atom and both X⁶ are independently (C₁-C₆)alkyl, the two (C₁-C₆)alkyl groups may be optionally joined and, together with the atom to which the two X⁶ groups are attached, form a 4- to 9- membered ring optionally having oxygen, sulfur or NX⁷ as a ring member;
X⁷ is hydrogen or (C₁-C₆)alkyl optionally substituted with hydroxy;
m for each occurrence is independently 0, 1 or 2;
with the provisos that:
1) X⁶ and X¹² cannot be hydrogen when attached to C(O) or S(O)₂ in the form C(O)X⁶, C(O)X¹², S(O)₂X⁶ or S(O)₂X¹²; and
2) when R⁶ is a bond then L is N(X²) and each r in the definition -(CH₂)ᵣ-L-(CH₂)ᵣ- is independently 2 or 3.

8. A use of claim 7 wherein the growth hormone secretagogue is a compound of Formula I-A 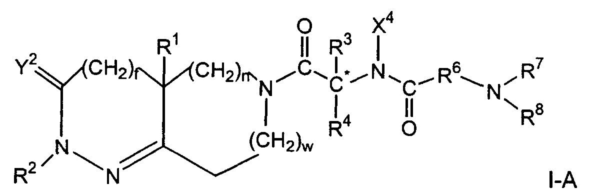 a racemic-diastereomeric mixture or an optical isomer of said compound or a pharmaceutically-acceptable salt or a prodrug thereof, or a tautomer thereof,
wherein
f is 0;
n is 0 and w is 2, or n is 1 and w is 1, or n is 2 and w is 0;
Y is oxygen or sulfur;
R¹ is hydrogen, -CN, -(CH₂)_{q}N(X⁶)C(O)X⁶, -(CH₂)_{q}N(X⁶)C(O)(CH₂)ₜ-A¹, -(CH₂)_{q}N(X⁶)SO₂(CH₂)ₜ-A¹, -(CH₂)_{q}N(X⁶)SO₂X⁶, -(CH₂)_{q}N(X⁶)C(O)N(X⁶)(CH₂)ₜ-A¹, -(CH₂)_{q}N(X⁶)C(O)N(X⁶)(X⁶), -(CH₂)_{q}C(O)N(X⁶)(X⁶), -(CH₂)_{q}C(O)N(X⁶)(CH₂)ₜ-A¹, -(CH₂)_{q}C(O)OX⁶, -(CH₂)_{q}C(O)O(CH₂)ₜ-A¹, -(CH₂)_{q}OX⁶, -(CH₂)_{q}OC(O)X⁶, (CH₂)_{q}OC(O)(CH₂)ₜ-A¹, -(CH₂)_{q}OC(O)N(X⁶)(CH₂)ₜ-A¹, -(CH₂)_{q}OC(O)N(X⁶)(X⁶), -(CH₂)_{q}C(O)X⁶, (CH₂)_{q}C(O)(CH₂)ₜ-A¹, -(CH₂)_{q}N(X⁶)C(O)OX⁶, -(CH₂)_{q}N(X⁶)SO₂N(X⁶)(X⁶), -(CH₂)_{q}S(O)ₘX⁶, -(CH₂)_{q}S(O)ₘ(CH₂)ₜ-A¹, -(C₁-C₁₀)alkyl, -(CH₂)ₜ-A¹, -(CH₂)_{q}-(C₃-C₇)cycloalkyl, -(CH₂)_{q}-Y¹-(C₁-C₆)alkyl, -(CH₂)_{q}-Y¹-(CH₂)ₜ-A¹ or -(CH₂)_{q}-Y¹-(CH₂)ₜ-(C₃-C₇)cycloalkyl;
where the alkyl and cycloalkyl groups in the definition of R¹ are optionally substituted with (C₁-C₄)alkyl, hydroxyl, (C₁-C₄)alkoxy, carboxyl, -CONH₂, -S(O)ₘ(C₁-C₆)alkyl, -CO₂(C₁-C₄)alkyl ester, 1H-tetrazol-5-yl or 1, 2 or 3 fluoro;
Y¹ is O, S(O)ₘ, -C(O)NX⁶-, -CH=CH-, -C≡C-, -N(X⁶)C(O)-, -C(O)O-, -OC(O)N(X⁶)- or -OC(O)-;
q is 0, 1, 2, 3 or 4;
t is 0, 1, 2 or 3;
said (CH₂)_{q} group and (CH₂)ₜ group may each be optionally substituted with hydroxyl, (C₁-C₄)alkoxy, carboxyl, -CONH₂, -S(O)ₘ(C₁-C₆)alkyl,
-CO₂(C₁-C₄)alkyl ester, 1H-tetrazol-5-yl, 1, 2 or 3 fluoro, or 1 or 2 (C₁-C₄)alkyl;
R² is hydrogen, (C₁-C₈)alkyl, -(C₀-C₃)alkyl-(C₃-C₈)cycloalkyl, -(C₁-C₄)alkyl-A¹ or A¹; where the alkyl groups and the cycloalkyl groups in the definition of R² are optionally substituted with hydroxyl, -C(O)OX⁶, -C(O)N(X⁶)(X⁶), -N(X⁶)(X⁶), -S(O)ₘ(C₁-C₆)alkyl, -C(O)A¹, -C(O)(X⁶), CF₃, CN or 1, 2 or 3 halogen;
R³ is A¹, (C₁-C₁₀)alkyl, -(C₁-C₆)alkyl-A¹, -(C₁-C₆)alkyl-(C₃-C₇)cycloalkyl, -(C₁-C₅)alkyl-X¹-(C₁-C₅)alkyl, -(C₁-C₅)alkyl-X¹-(C₀-C₅)alkyl-A¹ or -(C₁-C₅)alkyl-X¹-(C₁-C₅)alkyl-(C₃-C₇)cycloalkyl;
where the alkyl groups in the definition of R³ are optionally substituted with, -S(O)ₘ(C₁-C₆)alkyl, -C(O)OX³, 1, 2, 3, 4 or 5 halogens, or 1, 2 or 3 OX³; X¹ is O, S(O)ₘ, -N(X²)C(O)-, -C(O)N(X²)-, -OC(O)-, -C(O)O-, -CX²=CX²-, -N(X²)C(O)O-, -OC(O)N(X²)- or -C≡C-;
R⁴ is hydrogen, (C₁-C₆)alkyl or (C₃-C₇)cycloalkyl;
X⁴ is hydrogen or (C₁-C₆)alkyl or X⁴ is taken together with R⁴ and the nitrogen atom to which X⁴ is attached and the carbon atom to which R⁴ is attached and form a five to seven membered ring;
R⁶ is a bond or is 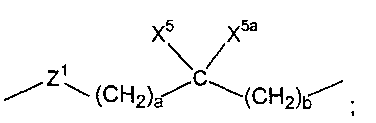
where a and b are independently 0, 1, 2 or 3;
X⁵ and X^{5a} are each independently selected from the group consisting of hydrogen, trifluoromethyl, A¹ and optionally substituted (C₁-C₆)alkyl;
the optionally substituted (C₁-C₆)alkyl in the definition of X⁵ and X^{5a} is optionally substituted with a substituent selected from the group consisting of A¹, OX², -S(O)ₘ(C₁-C₆)alkyl, -C(O)OX², (C₃-C₇)cycloalkyl, -N(X²)(X²) and -C(O)N(X²)(X²);
R⁷ and R⁸ are independently hydrogen or optionally substituted (C₁-C₆)alkyl;
where the optionally substituted (C₁-C₆)alkyl in the definition of R⁷ and R⁸ is optionally independently substituted with A¹, -C(O)O-(C₁-C₆)alkyl, -S(O)ₘ(C₁-C₆)alkyl, 1 to 5 halogens, 1 to 3 hydroxy, 1 to 3 -O-C(O)(C₁-C₁₀)alkyl or 1 to 3 (C₁-C₆)alkoxy; or
R⁷ and R⁸ can be taken together to form -(CH₂)ᵣ-L-(CH₂)ᵣ-;
where L is C(X²)(X²), S(O)ₘ or N(X²);
A¹ in the definition of R¹ is a partially saturated, fully saturated or fully unsaturated 4- to 8-membered ring optionally having 1 to 4 heteroatoms independently selected from the group consisting of oxygen, sulfur and nitrogen, a bicyclic ring system consisting of a partially saturated, fully unsaturated or fully saturated 5- or 6-membered ring, having 1 to 4 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
A¹ in the definition of R², R³, R⁶, R⁷ and R⁸ is independently (C₅-C₇)cycloalkenyl, phenyl or a partially saturated, fully saturated or fully unsaturated 4- to 8-membered ring optionally having 1 to 4 heteroatoms independently selected from the group consisting of oxygen, sulfur and nitrogen, a bicyclic ring system consisting of a partially saturated, fully unsaturated or fully saturated 5- or 6- membered ring, optionally having 1 to 4 heteroatoms independently selected from the group consisting of nitrogen, sulfur and oxygen, fused to a partially saturated, fully saturated or fully unsaturated 5- or 6- membered ring, optionally having 1 to 4 heteroatoms independently selected from the group consisting of nitrogen, sulfur and oxygen;
A¹ for each occurrence is independently optionally substituted, in one or optionally both rings if A¹ is a bicyclic ring system, with up to three substituents, each substituent independently selected from the group consisting of F, Cl, Br, I, OCF₃, OCF₂H, CF₃, CH₃, OCH₃, -OX⁶, -C(O)N(X⁶)(X⁶), -C(O)OX⁶, oxo, (C₁-C₆)alkyl, nitro, cyano, benzyl, -S(O)ₘ(C₁-C₆)alkyl, 1H-tetrazol-5-yl, phenyl, phenoxy, phenylalkyloxy, halophenyl, methylenedioxy, -N(X⁶)(X⁶), -N(X⁶)C(O)(X⁶), -SO₂N(X⁶)(X⁶), -N(X⁶)SO₂-phenyl, -N(X⁶)SO₂X⁶, -CONX¹¹X¹², -SO₂NX¹¹X¹², -NX⁶SO₂X¹², -NX⁶CONX¹¹X¹², -NX⁶SO₂NX¹¹X¹², -NX⁶C(O)X¹², imidazolyl, thiazolyl or tetrazolyl, provided that if A¹ is optionally substituted with methylenedioxy then it can only be substituted with one methylenedioxy;
where X¹¹ is hydrogen or optionally substituted (C₁-C₆)alkyl;
the optionally substituted (C₁-C₆)alkyl defined for X¹¹ is optionally independently substituted with phenyl, phenoxy, (C₁-C₆)alkoxycarbonyl, -S(O)ₘ(C₁-C₆)alkyl 1 to 5 halogens, 1 to 3 hydroxy, 1 to 3 (C₁-C₁₀)alkanoyloxy or 1 to 3 (C₁-C₆)alkoxy;
X¹² is hydrogen, (C₁-C₆)alkyl, phenyl, thiazolyl, imidazolyl, furyl or thienyl, provided that when X¹² is not hydrogen, X¹² is optionally substituted with one to three substituents independently selected from the group consisting of Cl, F, CH₃, OCH₃, OCF₃ and CF₃;
or X¹¹ and X¹² are taken together to form -(CH₂)ᵣ-L¹-(CH₂)ᵣ-;
where L¹ is C(X²)(X²), O, S(O)ₘ or N(X²);
r for each occurrence is independently 1, 2 or 3;
X² for each occurrence is independently hydrogen, optionally substituted (C₁-C₆)alkyl, or optionally substituted (C₃-C₇)cycloalkyl, where the optionally substituted (C₁-C₆)alkyl and optionally substituted (C₃-C₇)cycloalkyl in the definition of X² are optionally independently substituted with -S(O)ₘ(C₁-C₆)alkyl, -C(O)OX³, 1 to 5 halogens or 1-3 OX³;
X³ for each occurrence is independently hydrogen or (C₁-C₆)alkyl;
X⁶ is independently hydrogen, optionally substituted (C₁-C₆)alkyl, (C₂-C₆)halogenated alkyl, optionally substituted (C₃-C₇)cycloalkyl, (C₃-C₇)halogenatedcycloalkyl, where optionally substituted (C₁-C₆)alkyl and optionally substituted (C₃-C₇)cycloalkyl in the definition of X⁶ is optionally independently substituted by 1 or 2 (C₁-C₄)alkyl, hydroxyl, (C₁-C₄)alkoxy, carboxyl, CONH₂,-S(O)ₘ(C₁-C₆)alkyl, carboxylate (C₁-C₄)alkyl ester, or 1H-tetrazol-5-yl; or
when there are two X⁶ groups on one atom and both X⁶ are independently (C₁-C₆)alkyl, the two (C₁-C₆)alkyl groups may be optionally joined and, together with the atom to which the two X⁶ groups are attached, form a 4- to 9- membered ring optionally having oxygen, sulfur or NX⁷;
X⁷ is hydrogen or (C₁-C₆)alkyl optionally substituted with hydroxyl; and
m for each occurrence is independently 0, 1 or 2;
with the proviso that:
X⁶ and X¹² cannot be hydrogen when it is attached to C(O) or SO₂ in the form C(O)X⁶, C(O)X¹², SO₂X⁶ or SO₂X¹²; and
when R⁶ is a bond then L is N(X²) and each r in the definition -(CH₂)ᵣ-L-(CH₂)ᵣ- is independently 2 or 3.

9. A use of claim 8 wherein the compound, the prodrug thereof or the pharmaceutically acceptable salt of said compound or said prodrug, is selected from the group consisting of:
2-amino-N-(2-(3a-(R)-benzyl-2-methyl-3-oxo-2,3,3a,4,6,7-hexahydropyrazolo-[4,3-c]pyridin-5-yl)-1-(R)-benzyloxymethyl-2-oxo-ethyl)-isobutyramide;
2-amino-N-[2-(3a-(R)-benzyl-2-methyl-3-oxo-2,3,3a,4,6,7-hexahydropyrazolo[4,3-c]pyridin-5-yl)-1-(R)-benzyloxymethyl-2-oxo-ethyl]-isobutyramide, L-tartrate;
2-amino-N-(1-(R)-(2,4-difluoro-benzyloxymethyl)-2-oxo-2-(3-oxo-3a-(R)pyridin-2-ylmethyl-2-(2,2,2-trifluoro-ethyl)-2,3,3a,4,6,7-hexahydro-pyrazolo-[4,3-c]pyridin-5-yl)-ethyl)-2-methyl-propionamide; and
the (L)-(+)-tartaric acid salt of 2-amino-N-(1-(R)-(2,4-difluorobenzyloxymethyl)-2-oxo-2-(3-oxo-3a-(R)-pyridin-2-ylmethyl-2-(2,2,2-trifluoro-ethyl)-2,3,3a,4,6,7-hexahydro-pyrazolo-[4,3-c]pyridin-5-yl)-ethyl)-2-methyl-propionamide.

10. A use of claim 7 wherein the compound, the prodrug thereof or the pharmaceutically acceptable salt of said compound or said prodrug, is selected from the group consisting of:
2-amino-N-{1(R)-benzyloxymethyl-2-[1,3-dioxo-8a(S)-pyridin-2-ylmethyl-2-(2,2,2-trifluoro-ethyl)-hexahydro-imidazo[1,5-a]pyrazin-7-yl]-2-oxo-ethyl}-2-methylpropionamide; and
the (L)-(+)-tartaric acid salt of 2-amino-N-(1(R)-benzyloxymethyl-2-(1,3-dioxo-8a(S)-pyridin-2-ylmethyl-2-(2,2,2-trifluoro-ethyl)-hexahydro-imidazo[1,5-a]pyrazin-7-yl)-2-oxo-ethyl)-2-methyl-propionamide.

11. A use of claim 1 wherein the medicament further comprises a recombinant growth hormone or a growth hormone secretagogue selected from the group consisting of GHRP-6, GHRP-1, GHRP-2, hexarelin, growth hormone releasing factor, an analog of growth hormone releasing factor, IGF-I and IGF-II.

12. A use of claim 1 wherein the medicament further comprises an antidepressant, a prodrug thereof or a pharmaceutically acceptable salt of said antidepressant or said prodrug.

13. A use of claim 12 wherein said antidepressant is a selective serotonin reuptake inhibitor (SSRI) selected from citalopram, femoxetine, fluoxetine, fluvoxamine, indalpine, indeloxazine, milnacipran, paroxetine, sertraline, sibutramine and zimeldine, a prodrug of said SSRI or a pharmaceutically acceptable salt of said SSRI or said prodrug.

14. A use of claim 1 wherein the medicament further comprises an antipsychotic agent, a prodrug thereof or a pharmaceutically acceptable salt of said antipsychotic agent or said prodrug.

15. A use of claim 14 wherein the antipsychotic agent is chlorpromazine, haloperidol, clozapine, loxapine, molindone hydrochloride, thiothixene, olanzapine, ziprasidone hydrochloride, prochlorperazine, perphenazine, trifluoperazine hydrochloride or risperidone.

16. A use of claim 1 wherein the medicament further comprises an antianxiety agent, a prodrug thereof or a pharmaceutically acceptable salt of said antianxiety agent or said prodrug.

17. A use of claim 1 wherein the medicament further comprises a naturaceutic, a prodrug thereof or a pharmaceutically acceptable salt of said naturaceutic or said prodrug.

18. A use of claim 17 wherein the naturaceutic is ginko biloba, St. John's Wart, valerian or melatonin.
